# EUROPEAN PATENT SPECIFICATION

(11) **EP 4 493 566 B1**
(45) Date of publication and mention of the grant of the patent: **13.05.2026**
(21) Application number: 23715417.4
(22) Date of filing: 14.03.2023
(51) Int. Cl.: C07F 15/00, A61K 47/34, G01N 21/00, G01N 31/00, B82Y 15/00, G01N 31/22, A61K 47/24, A61K 9/51

(54) **COMPOUNDS FOR THE MEASUREMENT OF THE OXYGEN CONCENTRATION.**
VERBINDUNGEN ZUR MESSUNG DER SAUERSTOFFKONZENTRATION.
COMPOSÉS POUR LA MESURE DE LA CONCENTRATION EN OXYGÈNE.

(30) Priority: 14.03.2022 GB 202203502
(43) Date of publication of application: 22.01.2025
(73) Proprietor: Center for Neuromusculoskeletal Restorative Medicine Limited, New Territories (HK); Chen, Sijie, Sha Tin (HK); Ho, Po Yu, Sha Tin (HK)
(72) Inventor: CHEN, Sijie, Science Park, Sha Tin (HK); HO, Po Yu, Science Park, Sha Tin (HK)
(74) Representative: Matter IP Limited
(86) International application number: PCT/EP2023/056519
(87) International publication number: WO 2023/174956

(56) References cited:
- JP-A- 2015 101 570
- ZHANG MENG-YING ET AL: "Strategy for Enhancing Second-Order Nonlinear Optical Properties of the Pt(II) Dithienylethene Complexes: Substituent Effect, [pi]-Conjugated Influence, and Photoisomerization Switch", THE JOURNAL OF PHYSICAL CHEMISTRY A, vol. 117, no. 47, 13 November 2013 (2013-11-13), US, pages 12497 - 12510, XP093051156, ISSN: 1089-5639, DOI: 10.1021/jp4041265

## Description

### Field of Invention

The present invention relates to new compounds and uses of these compounds for determining oxygen levels in a sample medium.

### Background and Prior Art

Unlike cultured adherent cells that grow in monolayers, cells in tissues grow in three-dimensions which relies on diffusion of oxygen from the surrounding environment to interior regions (Pampaloni, F.; Reynaud, E. G.; Stelzer, E. H. K. The third dimension bridges the gap between cell culture and live tissue. Nat. Rev. Mol. Cell Biol. 2007, 8, 839-845). As such, physiological hypoxia is one of the challenges hindering tissue engineering and is associated with microenvironments in pathological conditions such as cancer and Ischemia (Semenza, Gregg L. Hypoxia-Inducible Factors in Physiology and Medicine. Cell 2012, 148, 399-408). The significance of the oxygen distribution in biology is well accepted, but not fully understood due to the lack of reliable or feasible tools for oxygen level monitoring and mapping.

Nowadays, there are mainly five categories of oxygen-sensing techniques, which have been exploited to study oxygen levels in different biological systems. They are 1) polarographic electrode detection; 2) radioisotope imaging; 3) resonance imaging; 4) haemoglobin-based oxygen saturation (StO₂) optical detection; and 5) luminescence-based oxygen sensing (Roussakis, E.; Li, Z.; Nichols, A. J.; Evans, C. L. Oxygen-Sensing Methods in Biomedicine from the Macroscale to the Microscale. Angew. Chem. Int. Ed. 2015, 54, 8340-8362).

Polarographic electrode detection makes use of Clark electrodes to measure the electric current generated at the cathode upon voltage-driven oxygen reduction, while the oxygen partial pressure (pO₂) is directly proportional to the electric current measured. However, this technique is limited to invasive point-to-point detection.

Radioisotope imaging makes use of positron emission tomography (PET) scanners and intake of short-lived radiolabeled tracers to monitor oxygen metabolism rate. This technique is non-invasive, but requires the handling of hazardous radioactive species and suffers from relatively low spatial resolution.

Resonance imaging exploits nuclear magnetic resonance (NMR) scanning to trace stable and non-hazardous exogenous/endogenous contrast agent(s) with respect to distinct nuclear resonance frequencies in three-dimensional space upon applying a magnetic field. This technique is non-invasive and suitable for body/organ scanning. However, the spatial resolution is rather limited.

Haemoglobin-based oxygen saturation (StO₂) optical detection relies on the optical absorption difference of the deoxy-/oxy-haemoglobin couple, in which the difference will reflect the blood oxygenation with the aid of a pulse oximeter. This method cannot quantify the pO₂ of blood but renders StO₂ of blood in a limited sensing depth (e.g. finger of human body).

Luminescence-based oxygen sensing utilizes a set of optical readout devices (e.g. luminescence spectrometers and fluorescence microscopes) and oxygen-responsive luminogens.

Among these methods, luminescence-based oxygen sensing is the most favorite and popular one for biological studies because of its good sensitivity, low hazard, simple operation and the possibility for high-resolution multidimensional analysis. Also, supporting facilities such as fluorescence microscopes are widely available. Luminescent indicators have, therefore, become a thresholding factor in the development of oxygen sensing/mapping technique for biological applications.

Many luminescent oxygen probes have been developed for oxygen sensing. The sensing mechanisms includes oxygen-dependent reactions and luminescence quenching by oxygen (Papkovsky, D. B.; Dmitriev, R. I. Biological detection by optical oxygen sensing. Chem. Soc. Rev. 2013, 42, 8700-8732).

For the reaction-based oxygen sensors, a quencher is usually linked to the sensor so that the sensor is dark in the original state. The quencher is either conjugated to the sensor through a linker (e.g. an azo bond), which undergoes oxygen-dependent cleavage by intracellular reductases, or the quencher (e.g. zwitterionic N-oxide functional group) itself is involved in an oxygen-dependent enzymatic reaction (Kiyose, K.; Hanaoka, K.; Oushiki, D.; Nakamura, T.; Kajimura, M.; Suematsu, M.; Nishimatsu, H.; Yamane, T.; Terai, T.; Hirata, Y.; Nagano, T. Hypoxia-Sensitive Fluorescent Probes for in Vivo Real-Time Fluorescence Imaging of Acute Ischemia. J. Am. Chem. Soc. 2010, 132, 15846-15848 and Xu, C.; Zou, H.; Zhao, Z.; Zhang, P.; Kwok, R. T. K.; Lam, J. W. Y.; Sung, H. H. Y.; Williams, I. D.; Tang, B. Z. A New Strategy toward "Simple" Water-Soluble AIE Probes for Hypoxia Detection. Adv. Funct. Mater. 2019, 29, 1903278). The quencher will be cleaved, or the quenching effect will be prohibited after the reduction reaction under hypoxic conditions. Therefore, these luminescent probes have a turn-on response under low oxygen conditions.

Researchers have also demonstrated the application of these probes in intracellular oxygen sensing. However, since the detection is highly dependent on the activity of the enzyme and these reactions are irreversible, the measurement is indirect, and these probes are not suitable for mapping the dynamic change of oxygen levels in the bio-systems.

Most oxygen sensors are based on the effect of the luminescence quenching of the probe. Dynamic quenching by oxygen is a photophysical process and thus is fully reversible. Phosphorescence has a long decay time, and the probability of collisional quenching in the excited state of phosphors is therefore very high (Schweitzer, C.; Schmidt, R. Physical Mechanisms of Generation and Deactivation of Singlet Oxygen. Chem. Rev. 2003, 103, 1685-1758). Phosphorescent probes are thus very sensitive to the oxygen level.

Due to their phosphorescence properties and oxygen sensitivity, luminescent transition metal complexes have been synthesized and used as a major group of indicators for oxygen sensing (Wang, X.-d.; Wolfbeis, O. S. Optical methods for sensing and imaging oxygen: materials, spectroscopies and applications. Chem. Soc. Rev. 2014, 43, 3666-3761). Their luminescence typically decreases with a shortened luminescence lifetime in the presence of oxygen. These metal complexes can be further functionalized with hydrophilic polymers or incorporated into nanoparticles to make them more compatible to the biosystems. These probes are sensitive and reversible. Most of them, however, are monochromatic, so that the uneven distribution of the probes in the complex biosystem can be misinterpreted as varied local analyte concentration.

Luminescence lifetime is less dependent on the probe concentration and may offer us more accurate estimation of the oxygen level. Luminescence lifetime microscopes are specialized and expensive pieces of equipment that are not widely accessible. Therefore, a reversible ratiometric luminescent probe with oxygen-sensitivity would be ideal for oxygen sensing in the fields of biology. To attempt to achieve such a probe, some researchers have conjugated an oxygen sensitive molecule (e.g., a metal complex) with non-oxygen sensitive fluorophore. Zheng et al. made a reversible ratiometric oxygen-sensor by conjugating an oxygen sensitive poly(N-vinylpyrrolidone) (PVP)-conjugated Ir(III) complex with a yellow-emissive Rhodamine B at the hydroxyl terminal group of PVP block through the isothiocyanate label group (Zheng, X.; Wang, X.; Mao, H.; Wu, W.; Liu, B.; Jiang, X. Hypoxia-specific ultrasensitive detection of tumours and cancer cells in vivo.

Nat. Commun. 2015, 6, 5834 and Zheng, X.; Tang, H.; Xie, C.; Zhang, J.; Wu, W.; Jiang, X. Tracking Cancer Metastasis In Vivo by Using an Iridium-Based Hypoxia-Activated Optical Oxygen Nanosensor. Angew. Chem. Int. Ed. 2015, 54, 8094-8099). The design principle for this kind of dual-chromophore probe is straightforward. Compounds used for the measurement of the oxygen concentration are disclosed in JP 2015 101570 A.

Recently researchers have also discovered a group of interesting iridium(III) complexes with dual-phosphorescence that have ratiometric responses to oxygen level (Zhang, K. Y.; Gao, P.; Sun, G.; Zhang, T.; Li, X.; Liu, S.; Zhao, Q.; Lo, K. K.-W.; Huang, W. Dual-Phosphorescent Iridium(III) Complexes Extending Oxygen Sensing from Hypoxia to Hyperoxia. J. Am. Chem. Soc. 2018, 140, 7827-7834). However, generally speaking, there are still very few oxygen-sensitive reversible ratiometric luminescent probes available so far, and the synthetic preparation of these probes requires tedious multi-step synthesis processes.

The development of oxygen-sensing probes is, therefore, of utmost importance, especially in the fields of regenerative medicine and cancer biology it is highly desirable to develop the oxygen-sensitive reversible ratiometric luminescent probe with a simple synthetic route and related customized sensing platforms for different oxygen-related biological studies.

The inventors have surprisingly developed a dual-emissive transition metal complex that functions as a ratiometric oxygen probe having a fluorescent green peak and a phosphorescence red peak. The complex can be easily synthesized and is free of photoluminescent self-quenching problems, which many conventional luminogens suffer from during fluorescence imaging. Also, the complex may be readily incorporated with a lipid-based nanomaterial (e.g. DSPE-mPEG2000) upon a typical nanoprecipitation protocol and can be fabricated into water-soluble and gas-diffusive nanoparticles-based sensor for cellular investigation. Importantly, the ratiometric signal (Red/Green signal ratio) against oxygen concentration can be established as a linear relationship with good sensitivity, that is independent on solvent conditions. Based on cellular staining studies, this complex shows promise as a qualitative and even quantitative tool for ratiometric oxygen mapping *in vitro* (i.e. HeLa cells) upon calibration curve establishments and normoxia/hypoxia-differentiation in a dynamic fashion.

The listing or discussion of an apparently prior-published document in this specification should not necessarily be taken as an acknowledgement that the document is part of the state of the art or is common general knowledge.

### Description of the Invention

### Compounds of the Invention

According to a first aspect of the invention there is provided a compound according to Formula (I): 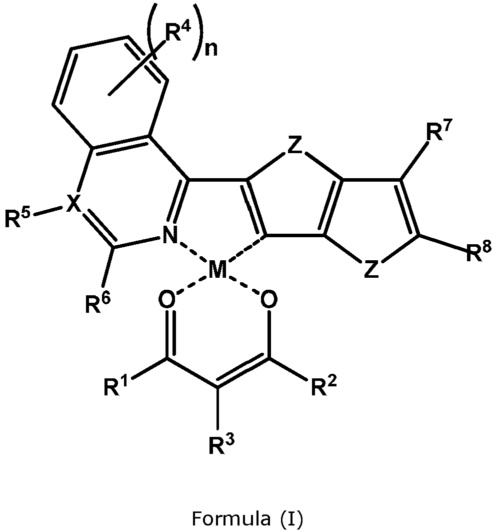 **wherein,**
M is a transition metal;
R¹, R², R³, R⁴, R⁵, R⁶, R⁷ and R⁸ are each independently selected from the group consisting of hydrogen, halogen, optionally substituted C₁-C₆ alkyl, optionally substituted C₁-C₆ haloalkyl, optionally substituted C₂-C₆ alkenyl, optionally substituted C₂-C₆ alkynyl, optionally substituted C₃-C₈ cycloalkyl, optionally substituted C₆-C₁₄ aryl, optionally substituted heteroaryl and -N(Y)₂;
n is 0 to 4;
Y is a C₁-C₆ alkyl, or a C₁-C₆ haloalkyl;
X is C or N; and
**Z is independently** selected from the list consisting of S, Se, and Te, which compounds are referred to herein after as "the compounds of the invention".

Compounds that may be included within the compounds of the invention include compounds according to Formula (I), wherein:
M is Pt or Pd;
R¹ and R² are independently selected from the group consisting of C₁-C₆ alkyl, C₁-C₆ haloalkyl, C₆-C₁₄ aryl and -N(Y)₂;
R³ is independently selected from the group consisting of hydrogen, C₁-C₆ alkyl, C₁-C₆ haloalkyl, C₆-C₁₄ aryl and -N(Y)₂;
R⁴ is selected from the group consisting of C₁-C₆ alkyl, C₁-C₆ haloalkyl, C₆-C₁₄ aryl, halogen and -N(Y)₂;
R⁵ and R⁶ are independently selected from the group consisting of hydrogen, C₁-C₆ alkyl, C₁-C₆ haloalkyl, C₆-C₁₄ aryl and -N(Y)₂;
R⁷ and R⁸ are independently selected from the group consisting of hydrogen, C₁-C₆ alkyl, C₁-C₆ haloalkyl, C₆-C₁₄ aryl and -N(Y)₂;
n is 0 to 4;
Y is a C₁-C₆ alkyl, or a C₁-C₆ haloalkyl;
X is C; and
Z is S.

Further compounds that may be included within the compounds of the invention include compounds according to Formula (I), wherein:
M is Pt or Pd;
R¹ and R² are independently selected from the group consisting of C₁-C₆ alkyl, C₁-C₆ haloalkyl, C₆-C₁₄ aryl and -N(Y)₂;
R³ is H;
R⁵ and R⁶ are H;
R⁷ and R⁸ are independently selected from the group consisting of hydrogen, C₁-C₆ alkyl, C₁-C₆ haloalkyl, C₆-C₁₄ aryl and -N(Y)₂;
n is 0;
Y is a C₁-C₆ alkyl, or a C₁-C₆ haloalkyl;
X is C; and
Z is S.

Particular compounds of the invention include compounds according to Formula (Ia) and Formula (Ib):

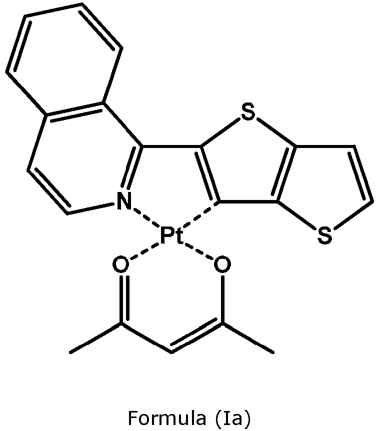

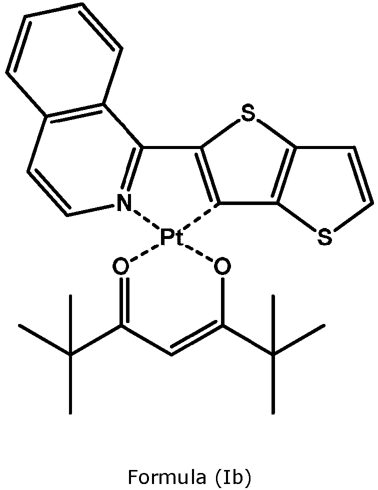

For the avoidance of doubt, compounds of the invention may exist as solids, and thus the scope of the invention includes all amorphous, crystalline and part crystalline forms thereof. Where compounds of the invention exist in crystalline and part crystalline forms, such forms may include solvates, which are included in the scope of the invention. Compounds of the invention may also exist in solution.

The present invention also embraces isotopically-labelled compounds of the invention, which are identical, but for the fact that one or more atoms are replaced by an atom having an atomic mass or mass number different from the atomic mass or mass number usually found in nature (or the most abundant one found in nature). All isotopes of any particular atom or element as specified herein are contemplated within the scope of the compounds of the invention. Hence, references to the compounds of the invention also includes deuterated compounds, i.e. in which one or more hydrogen atoms are replaced by the hydrogen isotope deuterium.

The term "transition metal" as used herein refers to an element whose atom has a partially filled d sub-shell, or which can give rise to cations with an incomplete d sub-shell. Particular transition metals that M may be chosen from include scandium, titanium, vanadium, chromium, manganese, iron, cobalt, nickel, copper, zinc, yttrium, zirconium, niobium, molybdenum, technetium, ruthenium, rhodium, palladium, silver, cadmium, lutetium, hafnium, tantalum, tungsten, rhenium, osmium, iridium, platinum, gold and mercury. For example, M may be platinum (Pt) or palladium (Pd).

The term "C₁₋₆ alkyl" when used alone or in combination with other terms, comprises a straight chain or branched C₁-C₆ alkyl which refers to monovalent alkyl groups having 1 to 6 carbon atoms. This term is exemplified by groups such as methyl, ethyl, n-propyl, i-propyl, n-butyl, s-butyl, i-butyl, t-butyl, n-pentyl, 1-ethylpropyl, 2-methylbutyl, 3-methylbutyl, 2,2-dimethylpropyl, n-hexyl, 2-methylpentyl, 3-methylpentyl, and 4-methylpentyl. Preferably, the term "C₁₋₆ alkyl" refers to C₁-C₆ alkyl, and more preferably C₁-C₄ alkyl, which, by analogy, refer respectively to monovalent alkyl groups having 1 to 5 carbon atoms, and monovalent alkyl groups having 1 to 4 carbon atoms.

The term "C₂-C₆ alkenyl" when used alone or in combination with other terms, comprises a straight chain or branched C₂-C₆ alkenyl. It may have any available number of double bonds in any available positions, and the configuration of the double bond may be the (E) or (Z) configuration. This term is exemplified by groups such as vinyl, allyl, isopropenyl, 1-propenyl, 2-methyl- 1-propenyl, 1-butenyl, 2-butenyl, 3-butenyl, 2-ethyl-1-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl, and the like. Preferably, the term "alkenyl" refers to C₂-C₅ alkenyl, such as a C₂-C₄ alkenyl.

The term "C₁₋C₆ haloalkyl" when used alone or in combination with other terms, comprises a straight chain or branched C₁-C₆ alkyl, wherein at least one hydrogen atom is replaced by a halogen atom.

The term " C₂-C₆ alkynyl" when used alone or in combination with other terms, comprises a straight chain or branched C₂-C₆ alkynyl. It may have any available number of triple bonds in any available positions. This term is exemplified by groups such as alkynyl groups that may have a carbon number of 2-6, and optionally a double bond, such as ethynyl (-C≡CH), 1-propynyl, 2-propynyl (propargyl: -CH₂C≡CH), 2-butynyl, and 2-pentene-4-ynyl. Particularly, these include C₂-C₅ alkynyl, such as C₂-C₄ alkynyl.

The term "C₃-C₈ cycloalkyl" refers to a saturated carbocyclic group of from 3 to 8 carbon atoms having a single ring (e.g. cyclohexyl) or multiple condensed rings (e.g. norbornyl). C₃-C₈ cycloalkyl includes cyclopentyl, cyclohexyl, and norbornyl.

The term "C₆-C₁₄ aryl" refers to an unsaturated aromatic carbocyclic group of from 6 to 14 carbon atoms having a single ring (e.g. phenyl) or multiple condensed rings (e.g. indenyl, naphthyl) and the term includes phenyl, naphthyl, anthryl, and phenanthrenyl.

The term "heteroaryl" refers to a monocyclic heteroaromatic, or a bicyclic or a tricyclic fused-ring heteroaromatic group. Particular examples of heteroaromatic groups include optionally substituted pyridyl, pyrrolyl, pyrimidinyl, furyl, thienyl, imidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, 1,3,4-triazinyl, 1,2,3-triazinyl, benzofuryl, [2,3-dihydro]benzofuryl, isobenzofuryl, benzothienyl, benzotriazolyl, isobenzothienyl, indolyl, isoindolyl, 3H-indolyl, benzimidazolyl, imidazo[1,2-a]pyridyl, benzothiazolyl, benzoxazolyl, quinolizinyl, quinazolinyl, pthalazinyl, quinoxalinyl, cinnolinyl, napthyridinyl, pyrido[3,4-b] pyridyl, pyrido[3,2-b]pyridyl, pyrido[4,3-b]pyridyl, quinolyl, isoquinolyl, tetrazolyl, 5,6,7,8-tetrahydroquinolyl, 5,6,7,8-tetrahydroisoquinolyl, purinyl, pteridinyl, carbazolyl, xanthenyl, and benzoquinolyl.

The term "halogen" refers to fluoro, chloro, bromo and iodo atoms.

### Process of Preparing the Compounds of the Invention

According to a second aspect of the invention there is provided a process for the preparation of a compound according to Formula (I), wherein the process comprises the steps of:
(i) reacting a compound according to Formula (II) with a compound according to Formula (III) to provide a compound according to Formula (IV) 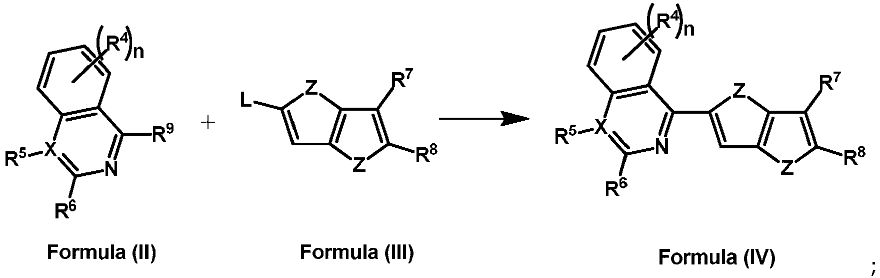
(ii) reacting the compound according to Formula (IV) with W₂M(R¹⁰)₄ to provide a compound according to Formula (V) 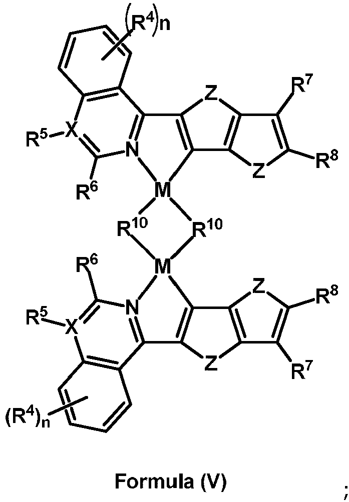 and
(iii) reacting a compound according to Formula (V) with a compound according to Formula (VI)

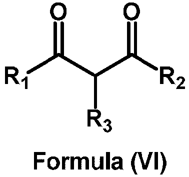

Wherein M, R¹, R², R³, R⁴, R⁵, R⁶, R⁷, R⁸, n, X and Z are as defined above in respect of the compounds of the invention;
R⁹ is a halogen;
R¹⁰ is a halogen;
L is boronic acid (-B(OH)₂) or a boronic ester; and
W is an alkali metal, which process is referred to herein after as "the process of the invention".
R⁹ may be fluoro, chloro, bromo or iodo, such as chloro.
R¹⁰ may be fluoro, chloro, bromo or iodo, such as chloro.
W may be lithium, sodium or potassium, such as potassium.

For the avoidance of doubt, M, R¹, R², R³, R⁴, R⁵, R⁶, R⁷, R⁸, n, X and Z are as defined above in all aspects in respect of the compounds of the invention.

For example:
M may be Pt or Pd;
R¹ and R² may independently be selected from the group consisting of C₁-C₆ alkyl, C₁-C₆ haloalkyl, C₆-C₁₄ aryl and -N(Y)₂;
R³ may be H;
R⁴ may be selected from the group consisting of consisting of C₁-C₆ alkyl, C₁-C₆ haloalkyl, C₆-C₁₄ aryl, halogen and -N(Y)₂;
R⁵ and R⁶ may be H;
R⁷ and R⁶ may be independently selected from the group consisting of hydrogen, C₁-C₆ alkyl, C₁-C₆ haloalkyl, C₆-C₁₄ aryl and -N(Y)₂;
n may be 0 to 4;
Y may be a C₁-C₆ alkyl, or a C₁-C₆ haloalkyl;
X may be C; and
Z may be S.

Such as:
M may be Pt or Pd;
R¹ and R² may be independently selected from the group consisting of C₁-C₆ alkyl, C₁-C₆ haloalkyl, C₆-C₁₄ aryl and -N(Y)₂;
R³ may be H;
R⁵ and R⁶ may be H;
R⁷ and R⁸ may be independently selected from the group consisting of hydrogen, C₁-C₆ alkyl, C₁-C₆ haloalkyl, C₆-C₁₄ aryl and -N(Y)₂;
n may be 0;
Y may be a C₁-C₆ alkyl, or a C₁-C₆ haloalkyl;
X may be C; and
Z may be S.

Step (i) of the process of the invention may be carried out under standard Suzuki cross coupling conditions, which means that L may represent a boronic acid (-B(OH)₂) or a boronic ester (e.g. N-methyliminodiacetic acid (MIDA), pinacol, neopentyl- or catechol boronates).

Step (i) of the process of the invention may be carried out in a binary solvent system, comprising an organic solvent. For example, the binary solvent system of step (i) may comprise from about 50 to about 90 % water and from about 10 to about 50 % organic solvent, such as from about 60 to about 80 % water and from about 20 to about 40 % organic solvent.

The organic solvent of step (i) may be tetrahydrofuran (THF), toluene, dioxane, dimethylformamide, ethanol, n-butanol, dimethoxymethane, ethylene glycol dimethyl ether, water or mixtures thereof.

Step (i) may be carried out in the presence of a carbonate salt, such as an alkali metal carbonate, for example potassium carbonate.

Step (i) may be carried out in the presence of a suitable base such as sodium hydride, sodium hydroxide, sodium carbonate, potassium carbonate, caesium carbonate, triethylamine, di-iso-propylamine or mixtures thereof.

Step (i) may also be carried out in the presence of a catalyst, such as [1,1'-bis(diphenylphosphino)ferrocene]dichloropalladium(II), palladium(II) acetylacetonate, bis(acetonitrile)dichloropalladium(II), bis(dibenzylideneacetone)palladium(0), palladium(II) trifluoroacetate, tris(dibenzylideneacetone)dipalladium(0), tetrakis(triphenylphosphine)palladium(0), palladium(II) acetate, dichlorobis(tricyclohexylphosphine)palladium(II), bis(triphenylphosphine)palladium(II) dichloride, bis(di-tert-butyl(4-dimethylaminophenyl)phosphine)dichloropalladium(II), [1,1'-bis(diphenylphosphino)ferrocene]dichloropalladium(II), [1,1'-bis(di-tert-butylphosphino)ferrocene]dichloropalladium(II), palladium(II) chloride or mixtures thereof.

Step (i) of the process of the invention may also be carried out at an elevated temperature (e.g. at the reflux temperature of the solvent that is employed), for example a temperature above 25°C, such as from about 25°C to about 90°C, for example about 50 to about 90°C.

Step (i) of the process of the invention may be carried out under microwave irradiation at above room temperature.

Following step (i), the product, being the compound according to Formula (IV), may be isolated. For example, the compound according to Formula (IV) may be extracted in a binary solvent system, such as a binary mixture of water and an organic solvent that is not miscible with water. The organic solvent that is not miscible with water may be ethyl acetate, dichloromethane or chloroform. Optionally the organic layer is dried using a drying agent, that is to say a drying agent may be added to the organic phase containing the compound of Formula (IV) to remove, or at least reduce, the amount of residual water in the organic phase. The drying agent may be a sulfate salt, such as sodium sulfate (Na₂SO₄) or magnesium sulfate (MgSO₄).

Following isolation, the organic solvent may be removed, at least partially, under reduced pressure.

Step (ii) of the process of the invention may be carried out in a binary solvent system comprising water and an organic solvent. For example, the binary solvent system of step (ii) may comprise from about 10 to about 50 % water and from about 50 to about 90 % of an organic solvent, such as from about 10 to about 30 % water and from about 70 to about 90 % of an organic solvent.

The organic solvent of step (ii) may be an alcohol, such as 2-ethoxyethanol or 2-methoxyethanol.

Step (ii) of the process of the invention may also be carried out at an elevated temperature, for example a temperature above 25°C, such as from about 50°C to about 150°C, for example about 80°C to about 120°C.

Following step (ii), the product, being the compound according to Formula (V), may be isolated. For example, the compound according to Formula (V) may be filtered from the binary solvent system. The solid product may be washed, for example washed with a mixture of water and ethanol. Furthermore, the resulting isolated solid may be dried, optionally under reduced pressure.

Step (iii) of the process of the invention may be carried out in an organic solvent, such as an alcohol containing organic solvent, for example 2-ethoxyethanol or 2-methoxyethanol.

Step (iii) of the process of the invention may also be carried out at an elevated temperature, for example a temperature above 25°C, such as from about 50°C to about 150°C, for example about 80°C to about 120°C.

Following step (iii), the product, being the compound according to Formula (I), may be isolated. For example, the compound according to Formula (I) may be extracted in a binary solvent system, such as a binary mixture of water and an organic solvent that is not miscible with water. The organic solvent that is not miscible with water may be chloroform (CHCl₃) or dichloromethane. Optionally the organic layer is dried using a drying agent, that is to say a drying agent may be added to the organic phase containing the compound of Formula (I) to remove, or at least reduce, the amount of residual water in the organic phase. The drying agent may be a sulfate salt, such as sodium sulfate (Na₂SO₄) or magnesium sulfate (MgSO₄).

### Loaded Nanoparticles

According to a third aspect of the invention there is provided a polymeric nanoparticle loaded with a compound according to Formula (I) as defined above in respect of the first aspect of the invention, which nanoparticles are referred to herein after as "the loaded nanoparticles of the invention".

By the term "loaded", we refer to polymeric nanoparticles containing the compound according to Formula I within the internal matrix of the nanoparticles. Although it is envisaged that the compound according to Formula I is within the internal matrix of the nanoparticles, this does not discount that some of the mass of the compound may also be on the surface of the nanoparticles.

The polymeric nanoparticle may be a nanolipid carrier. As used herein, the term "nanolipid carrier" refers to a nanoparticle formed from a polymer comprising a lipid. For example, the polymer may be a PEG-lipid conjugate.

The molecular weight of the PEG in the PEG-lipid conjugate may be in the range of from about 1000 to about 10,000, such as from about 1000 to about 6000, for example from about 2000 to about 5000.

The lipid of the PEG-lipid conjugate may be a phospholipid, such as 1, 2-distearoyl-sn-glycero-3-phosphoethanolamine (DSPE). Therefore, the PEG-lipid conjugate may be a DSPE-PEG conjugate.

The lipid of the PEG-lipid conjugate may further be a phospholipid-PEG conjugate (such as DSPE-mPEGX000 conjugates) or its bioconjugate derivatives, such as amine, carboxylic acid, maleimide or other derivatives.

The polymeric nanoparticle may be loaded with the compound according to Formula (I) in an amount of from about 0.1 % to about 10 w/w%, such as from about 0.1 to about 5 w/w% of the total solid content of the nanoparticle.

The polymeric nanoparticle may be present in an aqueous solution to provide a colloidal aqueous dispersion. That is to say in this aspect of the invention there is also provided a colloidal aqueous dispersion comprising a plurality of loaded nanoparticles of the invention.

The plurality of polymeric of nanoparticles in the colloidal aqueous dispersion may have a median diameter of from about 50 nm to about 1000 nm, such as from about 50 nm to about 500 nm, for example from about 50 nm to about 300 nm.

When a plurality of nanoparticles is present in a colloidal aqueous dispersion they may have a polydispersity index (PDI) of from about 0.1 to about 0.5, such as from about 0.2 to about 0.4.

When a plurality of nanoparticles are present in a colloidal aqueous dispersion they may have a zeta-potential of from about -20 to about -50 mV, such as from about -20 to about - 40 mV.

In the colloidal aqueous dispersion comprising a plurality of loaded nanoparticles the nanoparticles may be present in an amount of from about 5 to about 100 µg/mL, such as from about 10 to about 50 µg/mL.

For the purpose of fluorescence imagine experiments, the polymeric nanoparticle may be present in a freshly-prepared (e.g. prepared within the last 48 hours) aqueous solution, the concentration of which may be diluted by simply adding the necessary amount of pure water or buffer.

In this aspect of the invention there is also provided a method for preparing loaded polymeric nanoparticles, wherein the method comprises the steps of dispersing a compound according to Formula (I) in an organic solvent phase comprising a polymer, sonicating the solution, removing the organic solvent and redispersing the resulting solid in water.

For the avoidance of doubt, when used herein in relation to a specific value (such as an amount), the term "about" will be understood as indicating that such values may vary by up to 10 % (particularly, up to 5 %, such as up to 1 %) of the value defined. It is contemplated that, at each instance, such terms may be replaced with the notation "±10 %", or the like (or by indicating a variance of a specific amount calculated based on the relevant value). It is also contemplated that, at each instance, such terms may be deleted.

### Uses and Methods of Using the Compounds of the Invention

According to a fourth aspect of the invention there is provided the use of a compound according to Formula (I) as defined above, or a polymeric nanoparticle as defined above, for determining the concentration of oxygen in a medium.

The polymeric nanoparticle may be present in an aqueous solution to provide a colloidal aqueous dispersion. That is to say the use may be the use of a colloidal aqueous dispersion comprising a plurality of loaded nanoparticles of the invention for determining the concentration of oxygen in a medium.

The use may comprise a method comprising the steps of:
a. providing an aqueous solution comprising a plurality of loaded polymeric nanoparticles;
b. providing a sample in an aqueous medium;
c. adding the aqueous solution to the aqueous medium containing the sample; and
d. measuring the fluorescence spectrum of the compound according to Formula (I) contained in the loaded nanoparticles, which method is referred to herein as "the method of the invention".

The fluorescence spectrum of the compound according to Formula (I) may be measured by a fluorescence spectrophotometer or a confocal laser scanning microscope.

As used herein, the term "a plurality" refers to at least two.

The sample in the aqueous medium may be a biological sample, such as a plurality of cells. The cells may be cancer cells, such as HeLa cells, or fibroblast cells, such as NIH-3T3 cells.

The polymeric nanoparticles may be present in an aqueous solution in a concentration range of from about 1 to about 100 µM, such as from about 3 to about 60 µM, for example from about 5 to about 20 µM.

Compounds of Formula (I) may be synthesized according to the reported procedure, structurally characterized thereafter using standard methods and readily loaded into water-soluble phospholipid-based nanoparticles upon typical nanoprecipitation procedure, wherein the fresh nanoparticles may be further characterized by dynamic light scattering (DLS).

### Kit-Of-Parts

According to a fifth aspect of the invention there is provided a kit-of-parts comprising;
a. a solution comprising a compound according to Formula (I) as defined above, or a polymeric nanoparticle as defined above, or an aqueous composition as defined above; and
b. instructions for use of the kit in the method according to the method of the invention.

For the avoidance of doubt, the present invention and the uses of the newly developed kit for determining oxygen levels based on ratiometric photoluminescence signal for an aqueous or live cellular sample are fully supported by the experimental data provided herein from chemistry, photophysics, fluorescence imaging, and cell biology.

### Examples

The photophysics of the free compounds in tetrahydrofuran and the aqueous nanoparticles was studied in detail as outlined in this example section and both example species synthesized were found to demonstrate green fluorescence peak (oxygen-insensitive signal) and red phosphorescence peak (oxygen-sensitive signal) upon 500 nm photoexcitation. This sets the stage for the application of ratiometric oxygen sensing. Due to its high biocompatibility (i.e. relatively low cytotoxicity) with cells, the loaded nanoparticles may be utilized to stain cells overnight with an unspecified localization pattern, and the 2-channel confocal fluorescence imaging results demonstrated that the nanoparticles enabled the cellular oxygen mapping (etc. oxygen gradient illustration). In addition, the three-dimensional tumor spheroids of HeLa cells may also be penetrated and stained with the phospholipid-based nanoparticles and the natural oxygen gradient across the core-shell model was demonstrated.

The present invention has demonstrated that a series of precious metal complexes with relatively simple chemical structures, a shorter and straightforward synthetic route, and lower preparation cost may achieve the same or better photophysical properties to other ratiometric oxygen-sensing dyes or materials reported in the literature. The present invention will further facilitate the scientific investigations for the complicated relationships between oxygen and cell biology.

The invention is further illustrated, but not limited, by the following examples and accompanying figures, in which:
Figure 1 depicts (A) UV/Vis absorption of Pt(ttiq)(acac) in a CH₂Cl₂ solution. (B) Photoluminescence (PL) spectra of Pt(ttiq)(acac) in a CH₂Cl₂ solution in oxygen-free and ambient conditions under 500 nm photoexcitation. (C) PL spectra of the corresponding binary mixtures (water and tetrahydrofuran; 10 µM) of Pt(ttiq)(acac) in ambient condition under 500 nm photoexcitation. (D) PL spectra of the corresponding binary mixtures (water and tetrahydrofuran; 10 µM) of Pt(ttiq)(acac) in oxygen-free and ambient conditions under 500 nm photoexcitation. (E) PL spectra of the tetrahydrofuran solution (10 µM) of Pt(ttiq)(acac) in ambient condition under different photoexcitation wavelengths.

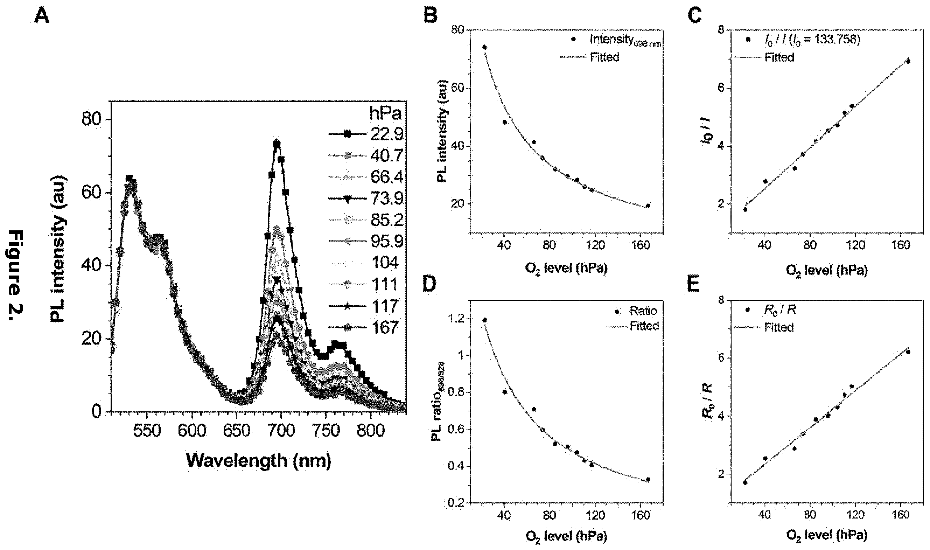

Figure 2 depicts (A) PL spectra of Pt(ttiq)(acac) (10 µM) in tetrahydrofuran in different oxygen partial pressures under 500 nm photoexcitation. (B) Plot of phosphorescence intensity against different oxygen partial pressures of the Pt(ttiq)(acac) (10 µM) in the tetrahydrofuran solution. (C) Plot of *I*₀ / *I* (phosphorescence intensity ratio; the estimated maximum phosphorescence intensity (*I*₀)) as a function of oxygen level. (D) Plot of PL intensity ratio (phosphorescence peak over fluorescence peak; the estimated maximum intensity ratio (*R*₀)) against different oxygen partial pressures of the Pt(ttiq)(acac) (10 µM) in the tetrahydrofuran solution. (E) Plot of *R*₀ / R (ratiometric signal ratio) in tetrahydrofuran as a function of oxygen level (i.e. a calibration curve for a tetrahydrofuran sample).

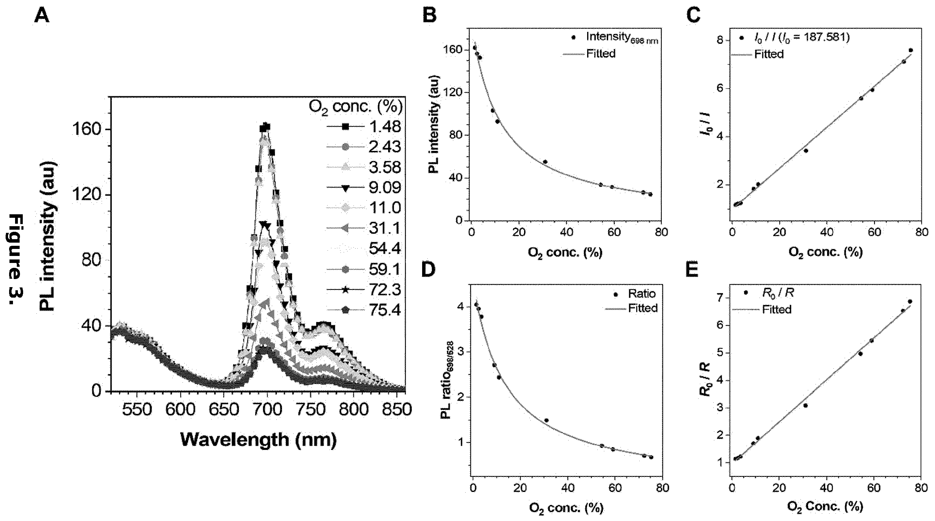

Figure 3 depicts (A) PL spectra of Pt(ttiq)(acac) nanoparticles (NPs) (29 µg mL⁻¹) in H₂O in different oxygen concentrations under 500 nm photoexcitation. (B) Plot of phosphorescence intensity against different oxygen concentrations of the Pt(ttiq)(acac) NPs (29 µg mL⁻¹) in H₂O. (C) Plot of *I*₀ / *I* (phosphorescence intensity ratio; the estimated maximum phosphorescence intensity (*I*₀)) as a function of oxygen level. (D) Plot of PL intensity ratio (phosphorescence peak over fluorescence peak; the estimated maximum intensity ratio (*R*₀)) against different oxygen concentrations of the Pt(ttiq)(acac) (29 µg mL⁻¹) in H₂O. (E) Plot of *R*₀/ R (ratiometric signal ratio) in H₂O as a function of oxygen level (i.e. a calibration curve for an aqueous sample).

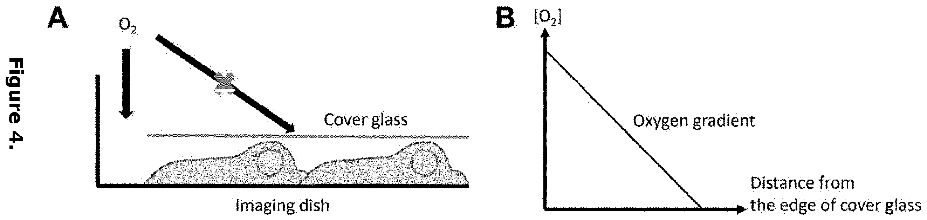

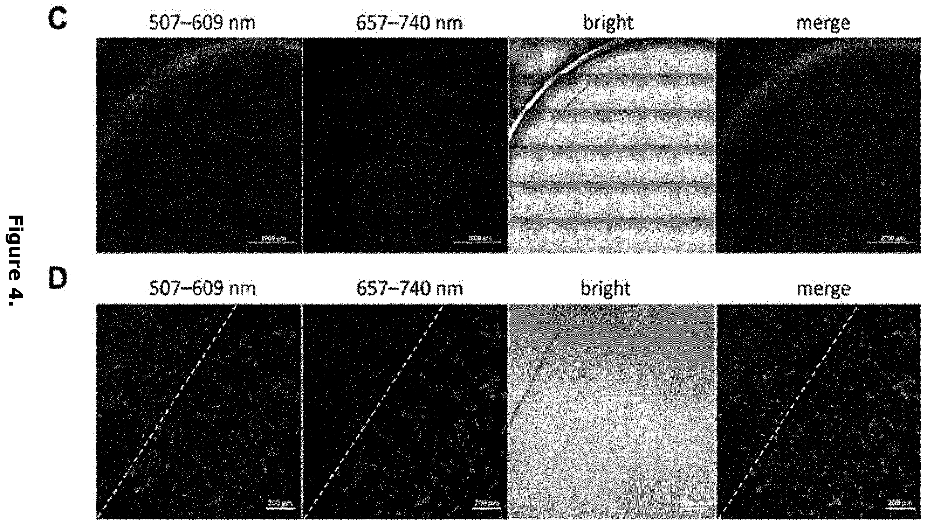

Figure 4 depicts (A) schematic diagram of the experimental set-up to induce an oxygen gradient across the overnight cultured 2D HeLa cells layer on an imaging dish. (B) Qualitative plot of oxygen level of the HeLa cells as a function of the distance from the edge of cover glass to the centre of the imaging dish. (C) Bright-field and fluorescence images (tile scans of the whole imaging dish) of the Pt(ttiq)(acac) NPs-stained HeLa cells (overnight staining, 29 µg mL⁻¹). Scale bar: 2000 µm. (D) Bright-field and fluorescence images (highlighting the area with the presence of oxygen gradient) of the Pt(ttiq)(acac) NPs-stained HeLa cells (overnight staining, 29 µg mL⁻¹). Scale bar: 200 µm.

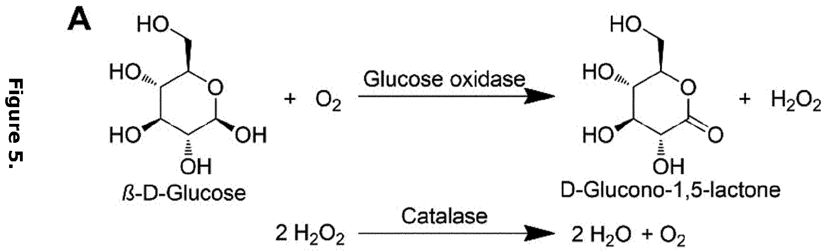

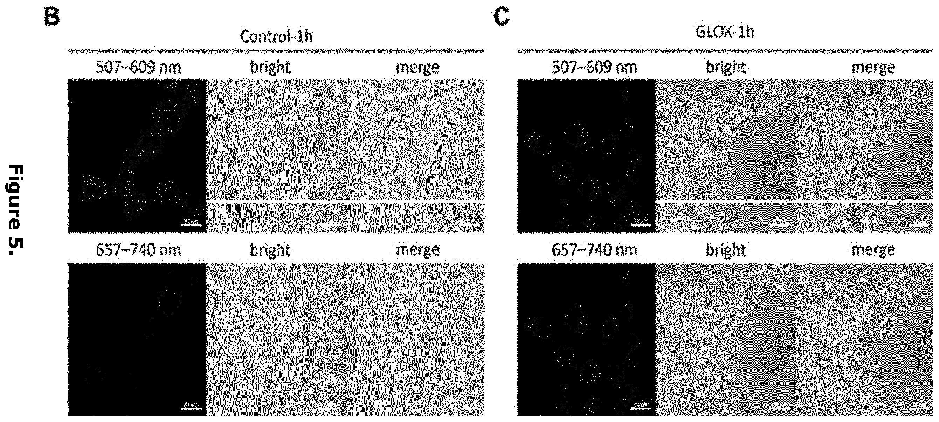

Figure 5 depicts (A) the active (bio)ingredients, enzymes and their catalytic reactions present in the STORM buffer (i.e. GLOX), which can act as an overall enzymatic oxygen scavenging buffer (developed by Zhong et al. (Huang, B.; Jones, S. A.; Brandenburg, B.; Zhuang X. Whole-cell 3D STORM Reveals Interactions between Cellular Structures with Nanometer-scale Resolution. Nat. Methods 2008, 5, 1047-1052) and it is specific for fluorescence cell imaging use). (B) Bright-field and fluorescence images of the Pt(ttiq)(acac) NPs-stained HeLa cells (overnight staining, 29 µg mL⁻¹) without the GLOX treatment (1 h). (C) Bright-field and fluorescence images of the Pt(ttiq)(acac) NPs-stained HeLa cells (overnight staining, 29 µg mL⁻¹) with the GLOX treatment (1 h). Scale bar: 20 µm.

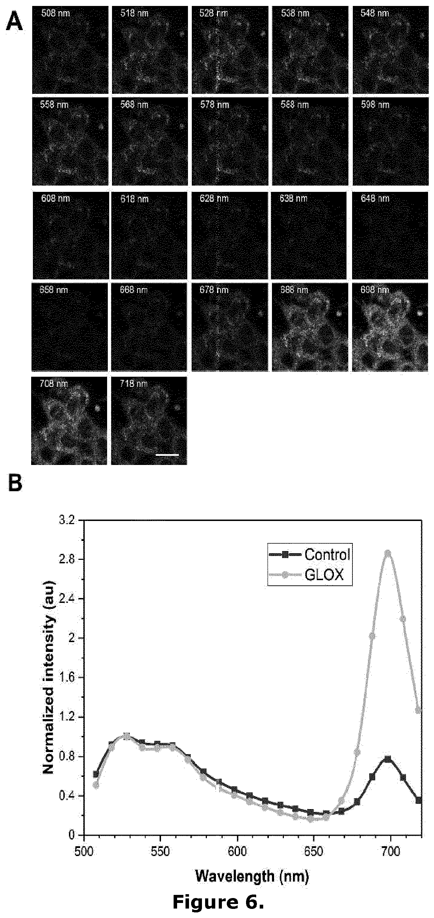

Figure 6 depicts (A) spectral imaging-resulted images (each channel is 10 nm apart) for the Pt(ttiq)(acac) NPs-stained HeLa cells (overnight staining, 29 µg mL⁻¹) *with* the GLOX treatment (1 h). Scale bar: 20 µm. (B) PL spectra obtained from the spectral images of the Pt(ttiq)(acac) NPs-stained HeLa cells (overnight staining, 29 µg mL⁻¹) with and without the GLOX treatment (1 h).

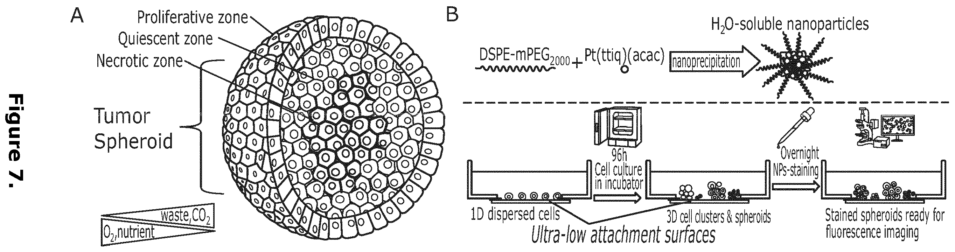

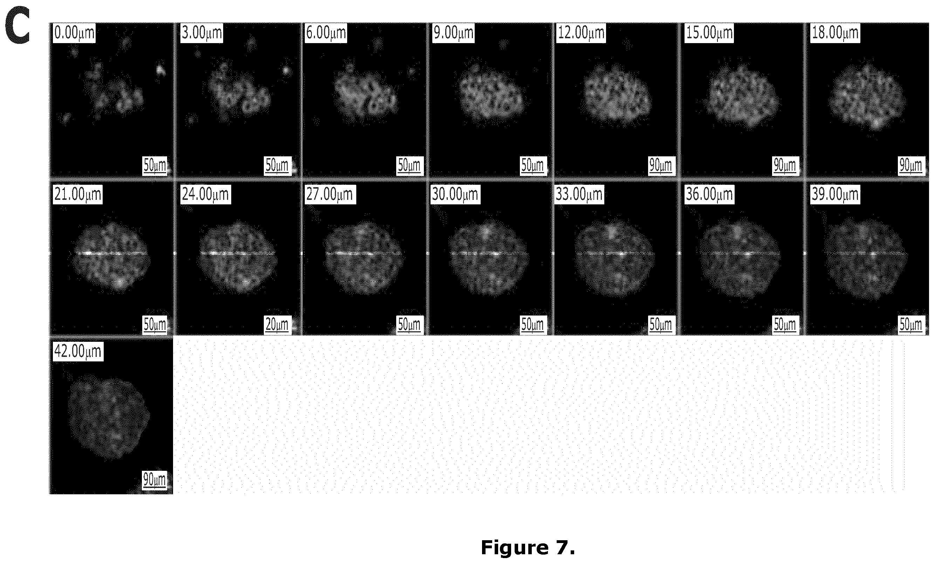

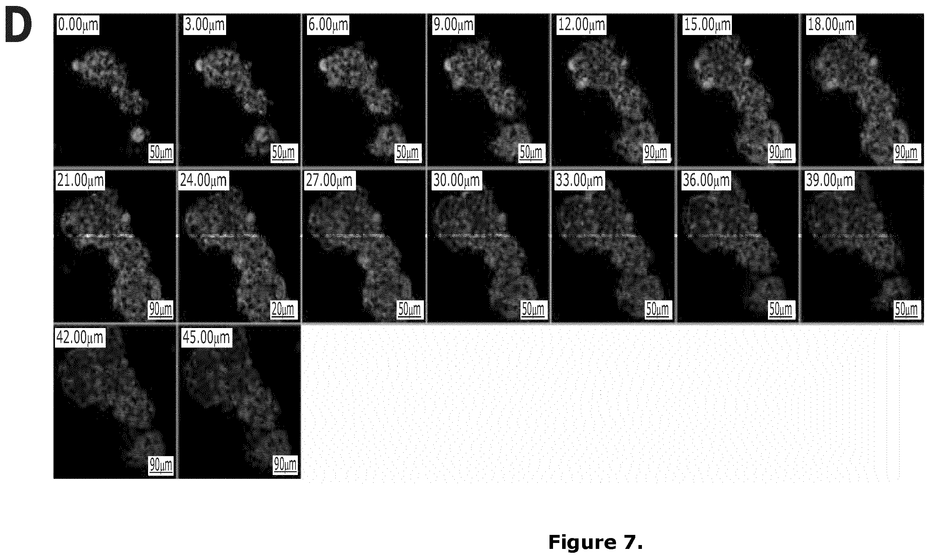

Figure 7 depicts (A) the zonation of a tumour spheroid and its metabolites' gradients (Costa, E. C.; Moreira, A. F.; de Melo-Diogo, D.; Gaspar, V. M.; Carvalho, M. P.; Correia, I. J. 3D Tumour Spheroids: an Overview on the Tools and Techniques Used for Their Analysis, Biotechnol. Adv., 2016, 34, 1427-1441). (B) The reagents to fabricate the Pt(ttiq)(acac) NPs through nanoprecipitation (upper); the flow of procedure to culture (96 h) and stain the HeLa tumour spheroids based on Pt(ttiq)(acac) NPs (lower). (C and D) Fluorescence images (Z-stacking scanning; each stack is 3 µm apart) of the Pt(ttiq)(acac) NPs-stained HeLa tumour spheroids (overnight staining, 29 µg mL⁻¹). Scale bar: 50 µm.

### Materials and reagents

All chemical reactions were performed under an inert nitrogen atmosphere with the use of a Schlenk line. Glassware was dried in oven prior to use. Commercially available reagents were used without purification. All the reagents for chemical synthesis were purchased from Tokyo Chemical Industry Co., Ltd (TCI), Sigma-Aldrich, Acros Organics and J&K Scientific. Dry solvents were purchased from the abovementioned companies and stored in the presence of activated 3 Å molecular sieves. All the reactions were monitored by thin-layer chromatography (TLC) with Merck pre-coated aluminium plates. Products were purified by column chromatography on silica gel (230-400 mesh from Merck or Davisil^{®} LC60Å 40-63 µm from W. R. Grace & Co.-Conn).

### Instruments for compounds' structural characterization and photophysical measurement

Proton and carbon NMR spectra were measured in CDCl₃ on a Bruker AVANCE III 400 MHz NMR Spectrometer and tetramethylsilane (TMS) was exploited as an internal standard for calibrating the chemical shift. Electrospray Ionization Quadrupole-Time-of-Flight (ESI-Q-TOF) mass spectrometry was performed on an Agilent 6540 Liquid chromatography - ESI-Q-TOF Mass Spectrometer. UV/Vis absorption spectroscopy was performed on a Molecular Devices SpectraMax M2e in different solutions at 293 K. Fluorescence emission spectra were taken from a LS-55 fluorescence spectrophotometer (PerkinElmer) at 293 K.

### Overall synthetic scheme

The scheme below depicts a schematic reaction for the preparation of an example compound according to the invention.

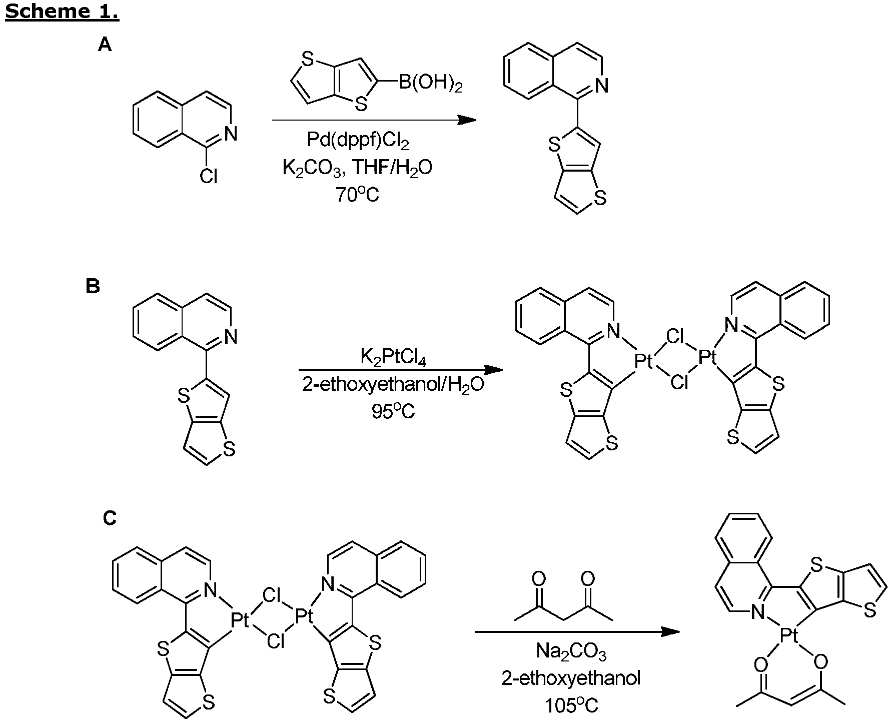

### Example 1 - Synthesis of Compounds of the Invention

### Synthesis of 1-(thieno[3,2-b]thiophen-2-yl)isoquinoline (ttiq)

To prepare 1-(thieno[3,2-b]thiophen-2-yl)isoquinoline, a mixture of 1-chloroisoquinoline (0.3 g, 1.834 mmol), thieno[3,2-b]thiophene-2-boronic acid (0.405 g, 2.2 mmol), Pd(PPh₃)₄ (63 mg, 0.055 mmol) and 2 M aqueous solution of K₂CO₃ (2.7 mL) in THF (30 mL) was heated to reflux under nitrogen atmosphere overnight. The reaction mixture was poured into water, followed by extraction using ethyl acetate. The organic layer was dried over anhydrous Na₂SO₄ and the solvent was then removed under reduced pressure.

The residue was purified by column chromatography on silica gel using a 1:9 mixture of ethyl acetate and hexane as eluent to afford 1-(thieno[3,2-b]thiophen-2-yl)isoquinoline as a slightly yellow shiny solid (334 mg, 1.248 mmol, 68 %). ¹H NMR (CDCl₃, 400 MHz): δ = 8.66-8.59 (m, 2H, Ar), 7.94-7.92 (m, 2H, Ar), 7.80-7.77 (m, 1H, Ar), 7.72-7.67 (m, 2H, Ar), 7.51 (d, 1H, J = 5.2 Hz, Ar), 7.36 (d, 1H, J = 5.2 Hz, Ar). ¹³C NMR (125 MHz, CDCl₃): δ = 153.05, 141.36, 141.16, 139.64, 137.41, 130.73, 128.72, 128.21, 127.43, 126.92, 126.07, 121.84, 120.36, 119.73 ppm (Ar). HRMS (MALDI-TOF, m/z): [(M+H)⁺] 268.0254; calcd for (C₁₅H₁₀NS₂⁺) 268.0249.

The schematic for the synthesis of 1-(thieno[3,2-b]thiophen-2-yl)isoquinoline can be seen in Scheme 1, part A above. For the avoidance of doubt, 1-(thieno[3,2-b]thiophen-2-yl)isoquinoline is referred to interchangeably throughout this specification by the abbreviation "ttiq" and has the following structure:

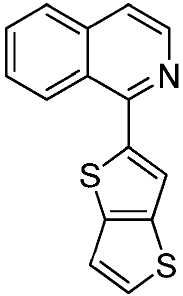

### Synthesis of platinum, di-µ-chlorobis[2-(1-isoquinolinyl-κN)(thieno[3,2-b]thien-3-yl-κC]di-

A mixture of 1-(thieno[3,2-b]thiophen-2-yl)isoquinoline (ttiq) (167 mg, 0.625 mmol) and K₂PtCl₄ (259 mg, 0.625 mmol) in a binary solvent system (2-ethoxyethanol (3 mL) and water (1 mL)) was stirred and heated to 95 °C under nitrogen atmosphere overnight. After cooling down, a red slurry was formed. The red participate was filtered out and washed with water and minimum amount of ethanol. The obtained solid residue after vacuum drying (231 mg, 0.231 mmol, 74 %) was used directly for the next reaction without further purification and characterization.

The schematic for the synthesis of platinum, di-µ-chlorobis[2-(1-isoquinolinyl-κ*N*)(thieno[3,2-b]thien-3-yl-κ*C*]di- can be seen in Scheme 1, part B above.

### Synthesis of platinum, (2,4-pentanedionato-κO², κO⁴)[2-(1-isoquinolinyl-κN)(thieno[3,2-b]thien-3-yl-κC]-,(SP-4-3)-

A mixture of Pt-Dimer (115 mg, 0.116 mmol), sodium carbonate (135 mg, 1.276 mmol) and acetylacetone (35 mg, 0.347 mmol) in 2-ethoxyethanol (3 mL) was stirred and heated to 105 °C under nitrogen atmosphere overnight. For the avoidance of doubt, acetylacetone is referred to interchangeably throughout this specification by the abbreviation "acac"

After cooling, the reaction mixture was poured into water, followed by extraction using chloroform. The organic layer was dried over anhydrous Na₂SO₄ and the solvent was then removed under reduced pressure. The residue was purified by column chromatography on silica gel using a 3:1 mixture of hexane and dichloromethane as eluent to give the product of part C of scheme 1 above, being platinum, (2,4-pentanedionato-κ*O*², κ*O*⁴)[2-(1-isoquinolinyl-κ*N*)(thieno[3,2-*b*]thien-3-yl-κ*C*]-,(*SP*-4-3)-;42 mg, 0.075 mmol, 32 %) as an orange solid.

For the avoidance of doubt the product of part C of scheme 1 above is referred to interchangeably throughout this specification by the abbreviation "Pt(ttiq)(acac)" and has the following structure:

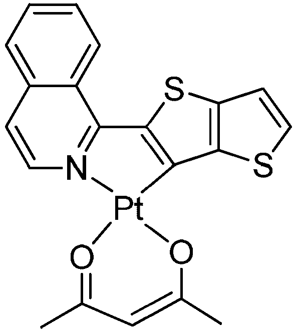

¹H NMR (CDCl₃, 400 MHz): δ = 8.75 (d, 1H, J = 6.4 Hz, Ar), 8.61 (d, 1H, J = 8.4 Hz, Ar), 7.79-7.60 (m, 4H, Ar), 7.28-7.23 (m, 2H, Ar), 5.55 (s, 1H, C(O)CH=C), 2.12 (s, 3H, alkyl), 2.07 ppm (s, 3H, alkyl). ¹³C NMR (125 MHz, CDCl₃): δ = 185.64, 184.04 (RC(O)R₁), 165.54, 144.80, 140.98, 139.73, 137.49, 133.26, 131.79, 128.06, 127.22, 126.97, 124.20, 118.78, 116.52, 102.84 (Ar), 28.35, 26.20 ppm (alkyl). HRMS (MALDI-TOF, m/z): [(M+H)⁺] 561.0266; calcd for (C₂₀H₁₆NO₂PtS₂⁺) 561.0265.

The schematic for the synthesis of platinum, (2,4-pentanedionato-κ*O*², κ*O*⁴)[2-(1-isoquinolinyl-κ*N*)(thieno[3,2-*b*]thien-3-yl-κ*C*]-,(*SP*-4-3)- can be seen in Scheme 1, part C above.

The UV/Vis absorption and photoluminescence (PL) spectra (excitation at 500 nm) of Pt(ttiq)(acac) (10 µM) in dichloromethane are exhibited in Figures 1A and 1B, respectively. The solution of Pt(ttiq)(acac) showed an increased maximum phosphorescence intensity at 698 nm when the oxygen content of the solution went down to zero by nitrogen purging (as is typical for phosphorescent Pt(II) cyclometalated complexes). However, Pt(ttiq)(acac), as a dual-emissive luminogen, also exhibited a constant PL peak at 528 nm (as a fluorescence) disregarding the oxygen content.

In view of the co-planar chemical structure of Pt(ttiq)(acac), it was interesting to look for any aggregation induced emission (AIE) or aggregation caused quenching (ACQ) property. In Figure 1C, the PL spectra of Pt(ttiq)(acac) exhibited were coming from the binary solution mixtures of water and tetrahydrofuran with different composition fractions. When the "bad" solvent (i.e. water) increased from 0 to 70 % in fraction and the metal complex started to form nano/micro-size aggregates, the red peak intensity increased gradually while the green peak intensity showed a gradual decline but without a complete quenching. This indicated that Pt(ttiq)(acac) was an AIE-active species in the region of 650-750 nm and that it was also an always-on PL panchromatic emitter. When the water fraction further increased to or above 80 %, the mixture formed clearly observable precipitates in the cuvette.

In order to observe whether these nanoaggregates were still readily quenched by oxygen, the PL spectra of the nanoaggregates' solution with a *f*_{water} of 60 % was compared before and after the deaeration. The results depicted in Figure 1D revealed that the nanoaggregates of Pt(ttiq)(acac) were still very responsive to the oxygen content and this indicated Pt(ttiq)(acac) could be loaded into biocompatible nanocarrier for oxygen-sensing applications.

In Figure 1E, the PL spectra of Pt(ttiq)(acac) (10 µM) in tetrahydrofuran upon different photoexcitation wavelengths were displayed and this showed that the PL profile of Pt(ttiq)(acac) was excitation wavelength dependent. 500 nm was the default excitation wavelength for Pt(ttiq)(acac).

The photoluminescence spectra of the Pt(ttiq)(acac) (10 µM) in tetrahydrofuran in different oxygen concentrations upon 500 nm photoexcitation is presented in Figure 2A. The phosphorescence peak intensity (698 nm) increases while the oxygen level of the solution decreases, whereas the fluorescence (528 nm) remains independent of the oxygen level. The oxygen quenching of the phosphorescence may be interpreted using Stern-Volmer relationship, in which (*I*₀ / *I =* 1 + *K*_{SV} ·[O₂]) where *I:* phosphorescence intensity; *I*₀: phosphorescence intensity at 0 % of oxygen; Ksv: quencher coefficient; [O₂]: oxygen concentration. The data of Figure 2A are fitted with this relationship in Figure 2B and thus the *I*₀ could be estimated from the equation of (y = 133.75801 / (1 + 0.0371 · x); adjusted *R*² = 0.98066). Therefore, a linear correlation of *I*₀ / *I* against [O₂] (y = 1.10962 + 0.03545 · x; adjusted *R*² = 0.99036) could be established as in Figure 2C. Mathematically, the Stern-Volmer relationship could be further derived to (*R*₀ / *R* = 1 + *K*_{SV} · [O₂]) whereas the fluorescence peak intensity is a constant, and thus the *R*₀ could be estimated from the equation of (y = 2.04177 / (1 + 0.03267 · x); adjusted *R*² = 0.97785) in Figure 2D. Similarly, a linear correlation of *R*₀ / R against [O₂] (y = 1.07595 + 0.03165 · x; adjusted *R*² = 0.98135) could be established as in Figure 2E. In short, the linear correlation between the ratiometric photoluminescence signal and oxygen concentration (%) obtained from Figure 2E may be utilized to analyze the oxygen content of a tetrahydrofuran sample without the concerns of concentration dependency and absolute intensity value.

The photoluminescence spectra of the loaded nanoparticles (29 µg mL⁻¹) in H₂O in different oxygen concentrations upon 500 nm photoexcitation are displayed in Figure 3A. The phosphorescence peak intensity (698 nm) increases while the oxygen level of the solution decreases, whereas the fluorescence (528 nm) remains independent of oxygen level. The oxygen quenching of the phosphorescence may be interpreted using Stern-Volmer relationship, in which (*I*₀ / *I =* 1 + *K*_{SV} · [O₂]) where *I:* phosphorescence intensity; *I*₀: phosphorescence intensity at 0 % of oxygen; *K*_{SV}: quencher coefficient; [O₂]: oxygen concentration. The data of Figure 3A are fitted with this relationship in Figure 3B and thus the *I*₀ could be estimated from the equation of (y = 187.58082 / (1 + 0.08449 · x); adjusted *R*² = 0.99487). Therefore, a linear correlation of *I*₀ / *I* against [O₂] (y = 0.97756 + 0.08536 · x; adjusted *R*² = 0.99786) could be established as in Figure 3C. Mathematically, the Stern-Volmer relationship may be further derived to (*R*₀ / *R* = 1 + Ksv · [O₂]) whereas the fluorescence peak intensity is a constant, and thus the *R*₀ could be estimated from the equation of (y = 4.61205 / (1 + 0.07422 · x); adjusted *R*² = 0.99646) in Figure 3D. Similarly, a linear correlation of *R*₀ / R against [O₂] (y = 0.95953 + 0.07641 · x; adjusted *R*² = 0.99702) could be established as in Figure 3E. In short, the linear correlation between the ratiometric photoluminescence signal and oxygen concentration (%) obtained from Figure 3E may be utilized to analyze the oxygen content of an aqueous sample without the concerns of concentration dependency and absolute intensity value.

### Example 2 - Fabrication of Loaded Nanoparticles and Characterisation

### Nanoparticle Fabrication

0.25 mL of platinum, (2,4-pentanedionato-κ*O*², κ*O*⁴)[2-(1-isoquinolinyl-κ*N*)(thieno[3,2-*b*]thien-3-yl-κ*C*]-,(*SP*-4-3)- in CH₂Cl₂ (2 mg/mL) and 1.667 mL of DSPE-mPEG2000 (15 mg/mL) were added into a 25 mL round bottom flask followed by mixing and mild sonicated for 3 min. The organic solvent was then removed under reduced pressure and a film of homogenous mixture (a doping layer with a doping % of 2 (w/w)) was formed upon vacuum drying (15 min) at room temperature. Under sonication, 10 mL of milli-Q H₂O was poured into the round bottom flask sharply and maintained the sonication for 5 min. This resulted in the formation of an aqueous nanoparticle solution which was filtered through a 0.45 µm syringe filter (PES (Polyethersulfone) membrane, Rephile). The size-filtered nanoparticle solution was then characterized using a dynamic light scattering (DLS) analyser (Zetasizer NanoZS, Malvern Panalytical) and the prepared fresh stock solution was used for cellular staining and calibration immediately each time.

### Calibration curves establishment against oxygen concentration (%) or partial pressure

The oxygen concentration (%) or partial pressure of solution samples were varied by O₂/N₂ gas-purging with a syringe needle and natural ventilation (or gradually adding sodium sulfite (Na₂SO₃) solution as an oxygen consuming chemical reagent). The instantaneous oxygen concentration (%) of aqueous samples (or oxygen partial pressure values of tetrahydrofuran solutions) at 293 K were measured using pre-calibrated FireSting^{®}-GO2 oxygen meter (PyroScience) coupled with a TDIP15 temperature probe (PyroScience) and a fiber optic sensor (OXR430-OI, or OXSOLV for organic solvent measurement) (PyroScience). The photoluminescence signals of the nano-sensor solution at different oxygen concentrations (%) (or partial pressures) were then measured by the fluorescence spectrophotometer and confocal laser scanning microscope. The calibration curves were established using the signal intensity at 528 nm and 698 nm from the PL spectra, and integrated signals' intensity from customized green channel (507-609 nm) and red channel (657-740 nm) from the CLSM images.

This overall experimental detail description is related to Figures 2 to 7.

### Example 3 - In Vitro Investigations

### 2D Cell culture

HeLa cells and NIH-3T3 cells were maintained in Dulbecco's Modified Eagle Medium (DMEM) supplemented with 10 % (Fetal Bovine Serum) FBS, 100 units/mL of penicillin, and 100 µg/mL of streptomycin in a 5 % CO₂ humidified incubator at 37 °C. Once the cells reached 80-90 % confluence, the cells were dissociated into single cells with 0.05 % trypsin-ethylenediaminetetraacetic acid (EDTA) at 37 °C for 5 min and passaged at a ratio of 1:6-1:19 in a new cell culture dish.

### Cytotoxicity of Pt(ttiq)(acac)/DSPE-mPEG2000 nanoparticles.

The influence of Pt(ttiq)(acac)/DSPE-mPEG2000 nanoparticles on the proliferation of HeLa/NIH-3T3 cells was assessed by a CCK-8 assay. Briefly, HeLa cells and NIH-3T3 cells were seeded in 96-well culture plates at a density of 5 × 10⁴ cells/well in a final volume of 200 µL. After culturing for 24 h, cells were treated with 0, 7.2, 14, 29, 43, 57, 86 µg mL⁻¹ of the Pt(ttiq)(acac) loaded nanoparticles for 24 h, respectively. Every sample was repeated three times. The cells were then washed with phosphate-buffered saline (PBS) three times. Finally, the cells were incubated in serum-free medium mixed with 20 % CCK-8 kit solution for 2 h at 37 °C. The absorbance was determined at 450 nm using a microplate reader (Molecular Devices SpectraMax M2e). The cell viability rate was calculated as percentage compared to the control cells: (absorbance of the treated wells) / (absorbance of the control wells) × 100 %.

### Cellular or spheroid fluorescence staining

For cellular fluorescence staining, HeLa cells were seeded on 35-mm glass-bottom dishes (Wuxi NEST Biotechnology Co., Ltd; NEST) for 48 h to allow cell attachment. For spheroid fluorescence staining, HeLa cells were culture in high-bioinert 35-mm glass-bottom dishes (ibidi GmbH) for 96 h to allow cellular aggregation formation. Subsequently, adherent cells or 3D spheroids were incubated with the Pt(ttiq)(acac)/DSPE-mPEG2000 nanoparticles (dispersed in fresh cell culture medium, 29 µg mL⁻¹, equivalent to ca. 10 µM of Pt(ttiq)(acac)) for 24 h. The cells or spheroids were then washed with PBS three times, cultured in complete DMEM medium without phenol red, and imaged by confocal microscopy. For the cover-glass-generated oxygen gradient experiment, a circular cover glass with a diameter of 30 mm (Paul Marienfeld) was placed carefully above the cells at the central position of the glass-bottom dishes after the staining and washing procedures.

The HeLa cells were further incubated overnight and the oxygen gradient was gradually formed around the cover glass edge (a schematic of this is shown in Figure 4A and Figure 4B).

An artificial oxygen gradient across 2D live cell layer is proposed to develop according to the experimental set up displayed in Figure 4A and the spatial correlation with the oxygen level is imaginatively configured in Figure 4B. In Figure 4C (2D tile scans), it was observed that the green channel (507-609 nm) demonstrated even signal in the HeLa cells under the cover glass, whereas the red channel (657-740 nm) displayed a stronger intensity in the HeLa cells near the centre of cover glass. This revealed that a hypoxic region was developed in the middle region under the cover glass and a circular normoxic region was observed near the edge of the cover glass under the cover glass. Under a higher magnification view, a clear oxygen gradient was visualized near the edge of cover glass in the merge image of both green and red channels (Figure 4D). This fully indicated that the staining loaded nanoparticles could differentiate hypoxic cells from normoxic cells with a good sensitivity.

### Preparation of GLOX solution and live cell STORM imaging buffer with 2-mercaptoethylamine (MEA)

Oxygen scavenging GLOX solution (250 µL), all components supplied by Millipore, was made up of 14 mg glucose oxidase, 50 µL catalase (17 mg/mL), and 200 µL buffer (10 mM Tris (pH 8.0) + 50 mM NaCl). The mixture was vortexed to dissolve glucose oxidase and then spun down at 14,000 rpm. The supernatant was subsequently pipetted for preparing the STORM imaging buffer. Per 700 µL of Stochastic Optical Reconstruction Microscopy (STORM) imaging buffer, 7 µL of the GLOX supernatant and 4.2 µL 1 M MEA (1 mL: 77 mg MEA + 1.0 mL 0.25 N aqueous HCl) were added into 690 µL live cell imaging buffer (DMEM + 75 mM HEPES ((4-(2-hydroxyethyl)-1-piperazineethanesulfonic acid) + 2 % glucose (w/w)). The mixture was vortexed gently and stored on ice right before adding into the stained cells for CLSM imaging use. Figures 5 and 6 represent the results.

In Figure 5A, the sum of the two enzymatic catalytic chemical reactions resulted in a net oxygen depletion present in the GLOX buffer. Upon the treatment of GLOX buffer toward the cultured 2D HeLa cells for one hour (Figure 5C), the turn-on of intracellular signal present in the red channel (657-740 nm) reflected that the oxygen depletion did happen intracellularly, and the hypoxia status could be successfully indicated upon the overnight staining of the loaded nanoparticles, after comparing the control group shown in Figure 5B. In the meanwhile, the turn-on signal from the green channel (507-609 nm) in both cellular samples further implied the loaded nanoparticles could be potentially exploited to quantify the oxygen content inside the HeLa cells based on the ratiometric signal readouts and a pre-established calibration curve. From the fluorescence pattern observed in both Figure 5B and 5C, the staining of the loaded nanoparticles was clearly not overlapped with any shaped intracellular organelles and instead resembled to the ordinary endosomes' staining.

The abovementioned observations and summaries could be further corroborated based on the fluorescence images of spectral imaging (GLOX treatment group versus control group) depicted in Figure 6A and the converted photoluminescence spectra shown in Figure 6B. Similarly, when the two green fluorescence peaks of both cellular samples at 528 nm were normalized, it was clear to figure out the intensified signal at 698 nm from the phosphorescence of the loaded nanoparticles in the experimental group. In addition, this verified that the freshly prepared GLOX buffer maintained its activity for at least one hour after treating the cells under the same conditions.

### Confocal Imaging

Confocal imaging was performed using the Zeiss LSM 880 confocal laser scanning microscope (CLSM) equipped with different objective lens (Plan-Apochromat 63×/1.4 NA oil objective lens, Plan-Apochromat 40x/1.3 NA oil objective lens, and Plan-Apochromat 10×/0.45 NA objective lens), a photo-multiplier tube, and a Gallium arsenide phosphide (GaAsP) detector driven by the ZEN software (Carl Zeiss). A 488-nm laser, a 488-nm beam splitter, a 507-609-nm emission filter, and a 657-740-nm emission filter were used for Pt(ttiq)(acac).

Spectral imaging was taken using a 32-channel GaAsP spectral detector driven by the Zeiss ZEN software (Carl Zeiss) in grayscale. A 488-nm laser and a 488-nm beam splitter were used. A range of 503-723 nm was detected at 10-nm resolution. Z-stack of spheroids was acquired at indicated intervals. Digital images were captured in grayscale, pseudo-coloured, and processed by ZEN software (ZEN 2.5 lite). Further image analysis was processed by ImageJ (NIH). Figure 6 represents the results.

### Fluorescent Oxygen Mapping of HeLa Spheroids

A natural oxygen gradient across 3D tumor spheroid is confirmed to develop according to the spatial correlation with the oxygen level displayed in Figure 7A. The protocol to formulate the loaded phospholipid-based nanoparticles and the cultured 3D tumor spheroid-staining protocol is demonstrated in Figure 7B. In both Figures 7C and 7D, the Z-stack scanning fluorescence images portrayed that the outermost layer of the tumor spheroid was dominated by the signal coming from the green channel (i.e. normoxic cells in proliferative zone) and the core layer of the tumor spheroid was dominated by the merged signals coming from both green and red channels (i.e. hypoxic cells in quiescent and necrotic zones). Overall, this visualized the presence of natural oxygen gradient in a tumor spheroid and also implied the potential application of the loaded nanoparticles for quantitative cellular or multicellular oxygen mapping, with a pre-established calibration curve under the confocal microscope.

## Claims

1. A compound according to formula (I): 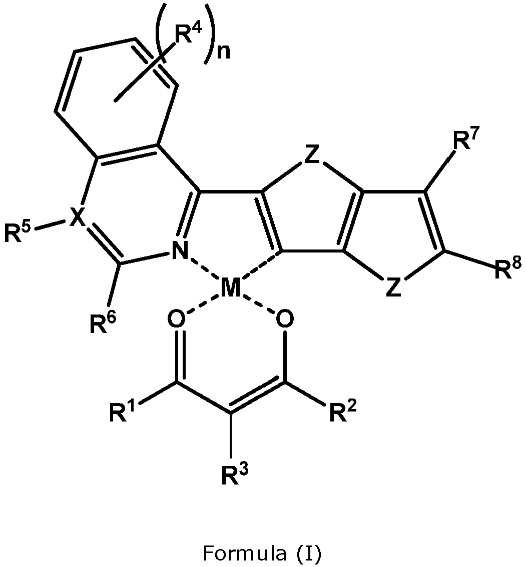 wherein,
M is a transition metal;
R¹, R², R³, R⁴, R⁵, R⁶, R⁷ and R⁸ are each independently selected from the group consisting of hydrogen, halogen, optionally substituted C₁-C₆ alkyl, C₁-C₆ haloalkyl, optionally substituted C₂-C₆ alkenyl, optionally substituted C₂-C₆ alkynyl, optionally substituted C₃₋₈ cycloalkyl, optionally substituted C₆-C₁₄ aryl, optionally substituted heteroaryl and -N(Y)₂;
n is 0 to 4;
Y is a C₁-C₆ alkyl, or a C₁-C₆ haloalkyl;
X is C or N; and
Z is independently selected from the list consisting of S, Se, and Te.

2. The compound according to Formula (I) as defined in Claim 1, wherein:
M is Pt or Pd;
R¹ and R² are independently selected from the group consisting of C₁-C₆ alkyl, C₁-C₆ haloalkyl, C₆-C₁₄ aryl and -N(Y)₂;
R³ is independently selected from the group consisting of hydrogen, C₁-C₆ alkyl, C₁-C₆ haloalkyl, C₆-C₁₄ aryl and -N(Y)₂;
R⁴ is selected from the group consisting of C₁-C₆ alkyl, C₁-C₆ haloalkyl, C₆-C₁₄ aryl, halogen and -N(Y)₂;
R⁵ and R⁶ are independently selected from the group consisting of hydrogen, C₁-C₆ alkyl, C₁-C₆ haloalkyl, C₆-C₁₄ aryl and -N(Y)₂;
R⁷ and R⁸ are independently selected from the group consisting of hydrogen, C₁-C₆ alkyl, C₁-C₆ haloalkyl, C₆-C₁₄ aryl and -N(Y)₂;
n is 0 to 4;
Y is a C₁-C₆ alkyl, or a C₁-C₆ haloalkyl;
X is C; and
Z is S.

3. The compound according to Formula (I) as defined in Claim 1 or Claim 2, wherein:
M is Pt or Pd;
R¹ and R² are independently selected from the group consisting of C₁-C₆ alkyl, C₁-C₆ haloalkyl, C₆-C₁₄ aryl and -N(Y)₂;
R³ is H;
R⁵ and R⁶ are H;
R⁷ and R⁸ are independently selected from the group consisting of hydrogen, C₁-C₆ alkyl, C₁-C₆ haloalkyl, C₆-C₁₄ aryl and -N(Y)₂;
n is 0;
Y is a C₁-C₆ alkyl, or a C₁-C₆ haloalkyl;
X is C; and
Z is S.

4. A compound as defined in any one of Claims 1 to 3, wherein the compound is according to Formula (Ia):

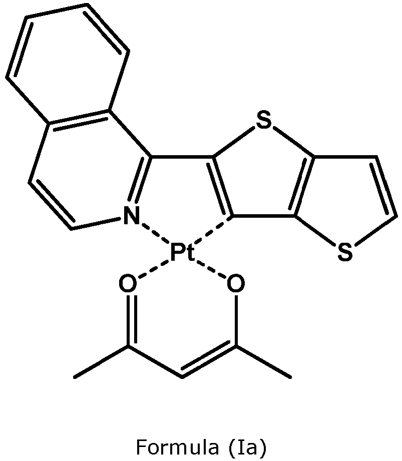

5. A compound as defined in any one of Claims 1 to 3, wherein the compound is according to Formula (Ib):

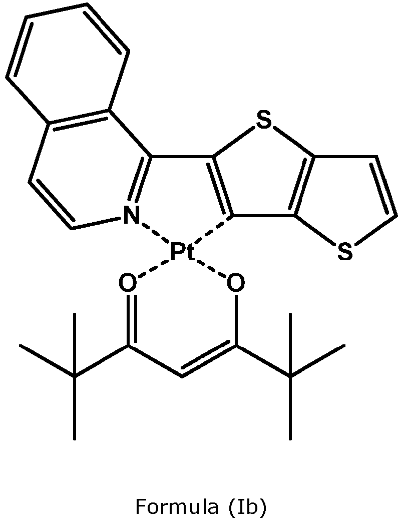

6. A process for the preparation of a compound according to Formula (I) as defined in any one of Claims 1 to 3, or a compound according to Formula (Ia) as defined in Claim 4, or a compound according to Formula (Ib) as defined in Claim 5, wherein the process comprises the steps of:
(i) reacting a compound according to Formula (II) with a compound according to Formula (III) to provide a compound according to Formula (IV), wherein L represents a boronic acid or boronic ester 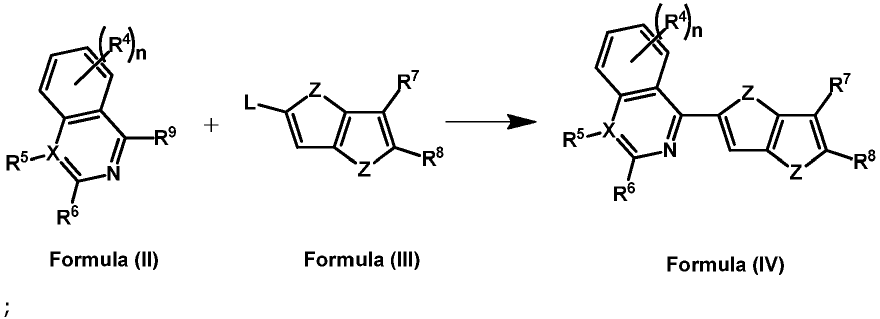
(ii) reacting the compound according to Formula (IV) with W₂M(R¹⁰)₄ to provide a compound according to Formula (V) 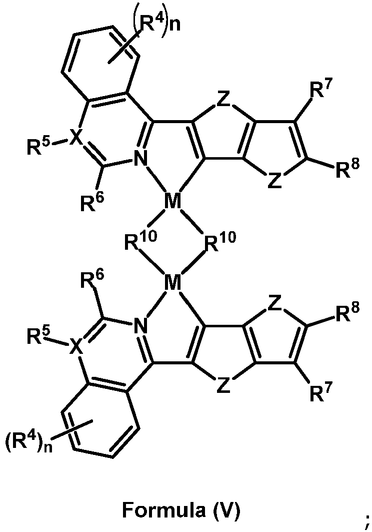 and
(iii) reacting a compound according to Formula (V) with a compound according to Formula (VI) 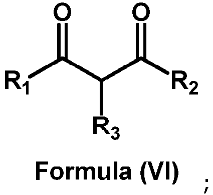
Wherein M, R¹, R², R³, R⁴, R⁵, R⁶, R⁷, R⁸, n, X and Z are as defined in any one of Claims 1 to 3;
R⁹ is a halogen;
R¹⁰ is a halogen; and
W is an alkali metal.

7. A polymeric nanoparticle loaded with a compound according to Formula (I) as defined in any one of Claims 1 to 3, or a compound according to Formula (Ia) as defined in Claim 4, or a compound according to Formula (Ib) as defined in Claim 5.

8. The polymeric nanoparticle according to Claim 7 wherein the polymeric nanoparticle is a nanolipid carrier, such as a PEG-lipid conjugate.

9. The polymeric nanoparticle according to Claim 8, wherein the molecular weight of the PEG in the PEG-lipid conjugate is about 1000 to about 10,000, about 1000 to about 6000, or about 2000 to about 5000.

10. The polymeric nanoparticle according to Claim 8, wherein the lipid of the PEG-lipid conjugate is a phospholipid, such as 1, 2-distearoyl-sn-glycero-3-phosphoethanolamine (DSPE).

11. The polymeric nanoparticle according to any one of Claims 7 to 10, wherein the compound according to Formula (I) is comprised in an amount of from about 0.1 w/w% to about 10 w/w% of the total solid content of the nanoparticle.

12. An aqueous composition comprising a plurality of polymeric nanoparticles according to any one of Claims 7 to 11.

13. Use of a compound according to Formula (I) as defined in any one of Claims 1 to 5, or a polymeric nanoparticle as defined in any one of Claims 7 to 11, or an aqueous composition as defined in Claim 12, for determining the concentration of oxygen in a medium.

14. A method of measuring the concentration of oxygen in a medium comprising using a compound according to Formula (I) as defined in any one of Claims 1 to 5, or a polymeric nanoparticle as defined in any one of Claims 7 to 11, or an aqueous composition according to Claim 12.

15. A kit-of-parts comprising;
a. a solution comprising a compound according to Formula (I) as defined in any one of Claims 1 to 5, or a polymeric nanoparticle as defined in any one of Claims 7 to 11, or an aqueous composition according to Claim 12; and
b. instructions for use of the kit in the method according to Claim 14.

## Patentansprüche

1. Verbindung gemäß Formel (I): 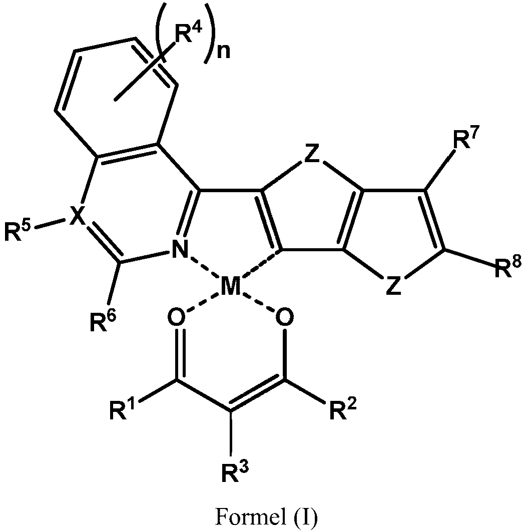 wobei
M ein Übergangsmetall ist;
R¹, R², R³, R⁴, R⁵, R⁶, R⁷ und R⁸ jeweils unabhängig ausgewählt sind aus der Gruppe bestehend aus Wasserstoff, Halogen, optional substituiertem C₁-C₆-Alkyl, C₁-C₆-Halogenalkyl, optional substituiertem C₂-C₆-Alkenyl, optional substituiertem C₂-C₆-Alkinyl, optional substituiertem C₃₋₈-Cycloalkyl, optional substituiertem C₆-C₁₄-Aryl, optional substituiertem Heteroaryl und -N(Y)₂;
n 0 bis 4 ist;
Y ein C₁-C₆-Alkyl oder ein C₁-C₆-Halogenalkyl ist;
Y C oder N ist; und
Z unabhängig ausgewählt ist aus der Liste bestehend aus S, Se und Te.

2. Verbindung gemäß Formel (I) nach Anspruch 1, wobei:
M Pt oder Pd ist;
R¹ and *R*² unabhängig aus der Gruppe ausgewählt sind, die aus C₁-C₆-Alkyl, C₁-C₆-Halogenalkyl, C₆-C₁₄-Aryl und -N(Y)₂ besteht;
R³ unabhängig ausgewählt ist aus der Gruppe bestehend aus Wasserstoff, C₁-C₆-Alkyl, C₁-C₆-Halogenalkyl, C₆-C₁₄-Aryl und -N(Y)₂;
R⁴ aus der Gruppe ausgewählt ist, die aus C₁-C₆-Alkyl, C₁-C₆-Halogenalkyl, C₆-C₁₄-Aryl, Halogen und -N(Y)₂ besteht;
R⁵ und R⁶ unabhängig ausgewählt sind aus der Gruppe bestehend aus Wasserstoff, C₁-C₆-Alkyl, C₁-C₆-Halogenalkyl, C₆-C₁₄-Aryl und -N(Y)₂;
R⁷ und R⁸ unabhängig ausgewählt sind aus der Gruppe bestehend aus Wasserstoff, C₁-C₆-Alkyl, C₁-C₆-Halogenalkyl, C₆-C₁₄-Aryl und -N(Y)₂;
n 0 bis 4 ist;
Y ein C₁-C₆-Alkyl oder ein C₁-C₆-Halogenalkyl ist;
X C ist; und
Z S ist.

3. Verbindung gemäß Formel (I) nach Anspruch 1 oder Anspruch 2, wobei:
M Pt oder Pd ist;
R¹ and R² unabhängig aus der Gruppe ausgewählt sind, die aus C₁-C₆-Alkyl, C₁-C₆-Halogenalkyl, C₆-C₁₄-Aryl und -N(Y)₂ besteht;
R³ H ist;
R⁵ und R⁶ H sind;
R⁷ und R⁸ unabhängig ausgewählt sind aus der Gruppe bestehend aus Wasserstoff, C₁-C₆-Alkyl, C₁-C₆-Halogenalkyl, C₆-C₁₄-Aryl und -N(Y)₂;
n 0 ist;
Y ein C₁-C₆-Alkyl oder ein C₁-C₆-Halogenalkyl ist;
X C ist; und
Z S ist.

4. Verbindung nach einem der Ansprüche 1 bis 3, wobei die Verbindung gemäß Formel (Ia) ist:

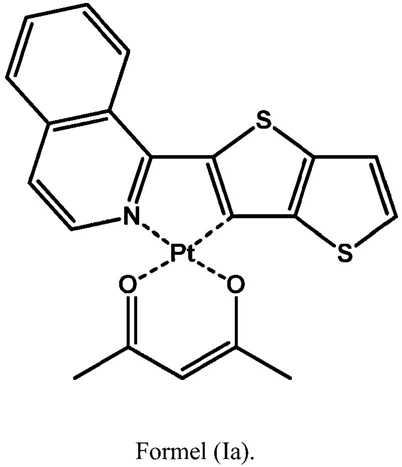

5. Verbindung nach einem der Ansprüche 1 bis 3, wobei die Verbindung gemäß Formel (Ib) ist:

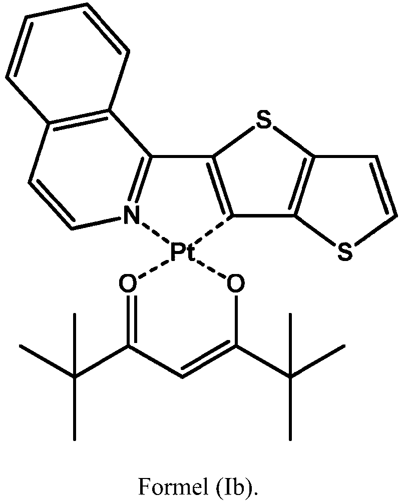

6. Verfahren zur Herstellung einer Verbindung gemäß Formel (I) nach einem der Ansprüche 1 bis 3 oder einer Verbindung gemäß Formel (Ia) nach Anspruch 4 oder einer Verbindung gemäß Formel (Ib) nach Anspruch 5, wobei das Verfahren die folgenden Schritte umfasst:
(i) Umsetzen einer Verbindung gemäß Formel (II) mit einer Verbindung gemäß Formel (III), um eine Verbindung gemäß Formel (IV) bereitzustellen, wobei L eine Boronsäure oder einen Boronsäureester darstellt 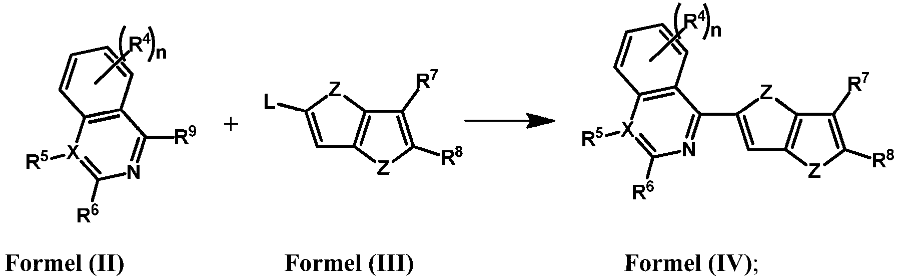
(ii) Umsetzen der Verbindung gemäß Formel (IV) mit W₂M(R¹⁰)₄, um eine Verbindung gemäß Formel (V) bereitzustellen 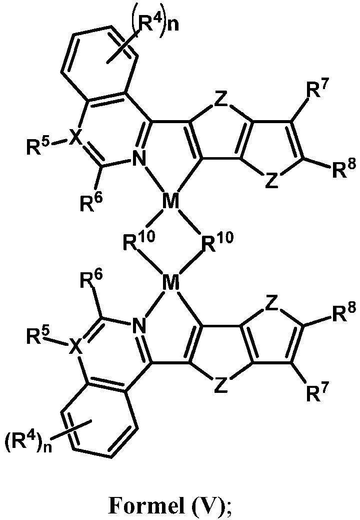 und
(iii) Umsetzen einer Verbindung gemäß Formel (V) mit einer Verbindung gemäß Formel (VI) 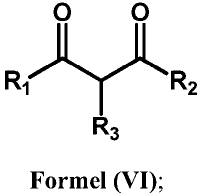
Wobei M, R¹, R², R³, R⁴, R⁵, R⁶, R⁷, R⁸, n, X und Z wie in einem der Ansprüche 1 bis 3 definiert sind;
R⁹ ein Halogen ist;
R¹⁰ ein Halogen ist; und
W ein Alkalimetall ist.

7. Polymeres Nanopartikel, das mit einer Verbindung gemäß Formel (I) nach einem der Ansprüche 1 bis 3 oder einer Verbindung gemäß Formel (Ia) nach Anspruch 4 oder einer Verbindung gemäß Formel (Ib) nach Anspruch 5 beladen ist.

8. Polymeres Nanopartikel nach Anspruch 7, wobei das polymere Nanopartikel ein Nanolipid-Träger, wie etwa ein PEG-Lipid-Konjugat, ist.

9. Polymeres Nanopartikel nach Anspruch 8, wobei das Molekulargewicht des PEG in dem PEG-Lipid-Konjugat etwa 1000 bis etwa 10.000, etwa 1000 bis etwa 6000 oder etwa 2000 bis etwa 5000 beträgt.

10. Polymeres Nanopartikel nach Anspruch 8, wobei das Lipid des PEG-Lipid-Konjugats ein Phospholipid, wie etwa 1,2-Distearoyl-sn-glycero-3-phosphoethanolamin (DSPE) ist.

11. Polymeres Nanopartikel nach einem der Ansprüche 7 bis 10, wobei die Verbindung gemäß Formel (I) in einer Menge von etwa 0,1 Gew.-% bis etwa 10 Gew.-% des gesamten Feststoffgehalts des Nanopartikels umfasst ist.

12. Wässrige Zusammensetzung, umfassend eine Vielzahl von polymeren Nanopartikeln nach einem der Ansprüche 7 bis 11.

13. Verwendung einer Verbindung gemäß Formel (I) nach einem der Ansprüche 1 bis 5 oder eines polymeren Nanopartikels nach einem der Ansprüche 7 bis 11 oder einer wässrigen Zusammensetzung nach Anspruch 12 zum Bestimmen der Konzentration von Sauerstoff in einem Medium.

14. Verfahren zum Messen der Konzentration von Sauerstoff in einem Medium, umfassend Verwenden einer Verbindung gemäß Formel (I) nach einem der Ansprüche 1 bis 5 oder eines polymeren Nanopartikels nach einem der Ansprüche 7 bis 11 oder einer wässrigen Zusammensetzung nach Anspruch 12.

15. Teilesatz, umfassend;
a. eine Lösung, umfassend eine Verbindung gemäß Formel (I) nach einem der Ansprüche 1 bis 5 oder ein polymeres Nanopartikel nach einem der Ansprüche 7 bis 11 oder eine wässrige Zusammensetzung nach Anspruch 12; und
b. Anweisungen zur Verwendung des Satzes in dem Verfahren nach Anspruch 14.

## Revendications

1. Composé selon la formule (I) : 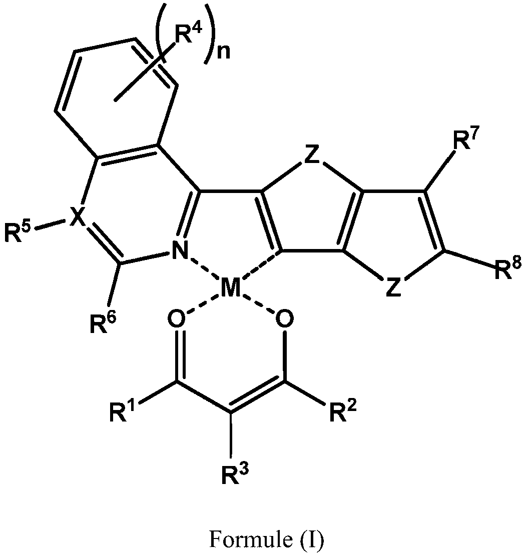 dans lequel,
M est un métal de transition ;
R¹, R², R³, R⁴, R⁵, R⁶, R⁷ et R⁸ sont chacun indépendamment choisis dans le groupe constitué par un hydrogène, un halogène, un alkyle en C₁-C₆ éventuellement substitué, un halogénoalkyle en C₁-C₆, un alcényle en C₂-C₆ éventuellement substitué, un alcynyle en C₂-C₆ éventuellement substitué, un cycloalkyle en C₃-C₈ éventuellement substitué, un aryle en C₆-C₁₄ éventuellement substitué, un hétéroaryle éventuellement substitué et -N(Y)₂ ;
n vaut 0 à 4 ;
Y est un alkyle en C₁-C₆ ou un halogénoalkyle en C₁-C₆ ;
X est C ou N ; et
Z est indépendamment choisi dans la liste constituée de S, Se et Te.

2. Composé selon la Formule (I) tel que défini dans la revendication 1, dans lequel :
M est Pt ou Pd;
R¹ et R² sont choisis indépendamment dans le groupe constitué par un alkyle en C₁-C₆, un halogénoalkyle en C₁-C₆, un aryle en C₆-C₁₄ et -N(Y)₂ ;
R³ est indépendamment choisi dans le groupe constitué par un hydrogène, un alkyle en C₁-C₆, un halogénoalkyle en C₁-C₆, un aryle en C₆-C₁₄ et -N(Y)₂ ;
R⁴ est choisi dans le groupe constitué par un alkyle en C₁-C₆, un halogénoalkyle en C₁-C₆, un aryle en C₆-C₁₄, un halogène et -N(Y)₂ ;
R⁵ et R⁶ sont indépendamment choisis dans le groupe constitué par un hydrogène, un alkyle en C₁-C₆, un halogénoalkyle en C₁-C₆, un aryle en C₆-C₁₄ et -N(Y)₂ ;
R⁷ et R⁸ sont indépendamment choisis dans le groupe constitué par un hydrogène, un alkyle en C₁-C₆, un halogénoalkyle en C₁-C₆, un aryle en C₆-C₁₄ et -N(Y)₂ ;
n vaut 0 à 4 ;
Y est un alkyle en C₁-C₆ ou un halogénoalkyle en C₁-C₆ ;
X est C ; et
Z est S.

3. Composé selon la Formule (I) tel que défini dans la revendication 1 ou la revendication 2, dans lequel :
M est Pt ou Pd ;
R¹ et R² sont choisis indépendamment dans le groupe constitué par un alkyle en C₁-C₆, un halogénoalkyle en C₁-C₆, un aryle en C₆-C₁₄ et -N(Y)₂ ;
R³ est H ;
R⁵ et R⁶ sont H ;
R⁷ et R⁸ sont indépendamment choisis dans le groupe constitué par un hydrogène, un alkyle en C₁-C₆, un halogénoalkyle en C₁-C₆, un aryle en C₆-C₁₄ et -N(Y)₂ ;
n vaut 0 ;
Y est un alkyle en C₁-C₆ ou un halogénoalkyle en C₁-C₆ ;
X est C ; et
Z est S.

4. Composé selon l'une quelconque des revendications 1 à 3, ledit composé répondant à la Formule (Ia) :

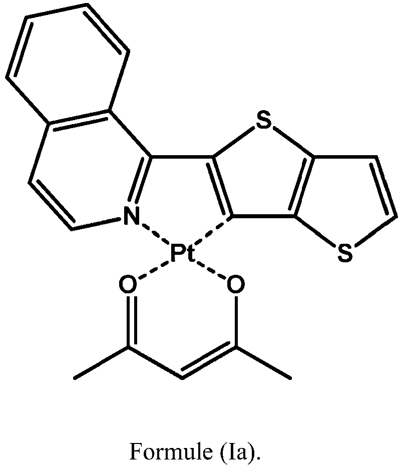

5. Composé selon l'une quelconque des revendications 1 à 3, ledit composé répondant à la Formule (Ib) :

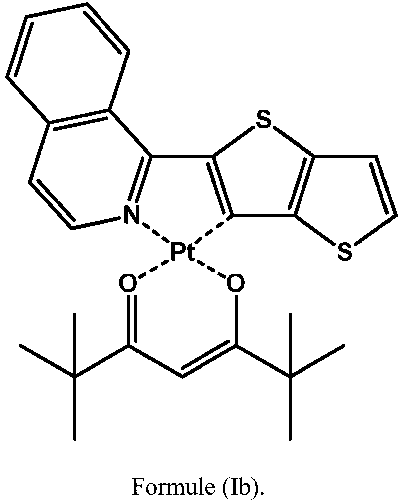

6. Processus permettant la préparation d'un composé selon la Formule (I) tel que défini dans l'une quelconque des revendications 1 à 3, ou d'un composé selon la Formule (Ia) tel que défini dans la revendication 4, ou d'un composé selon la Formule (Ib) tel que défini dans la revendication 5, ledit processus comprenant les étapes de :
(i) réaction d'un composé selon la Formule (II) avec un composé selon la Formule (III) pour obtenir un composé selon la Formule (IV), où L représente un acide boronique ou un ester boronique 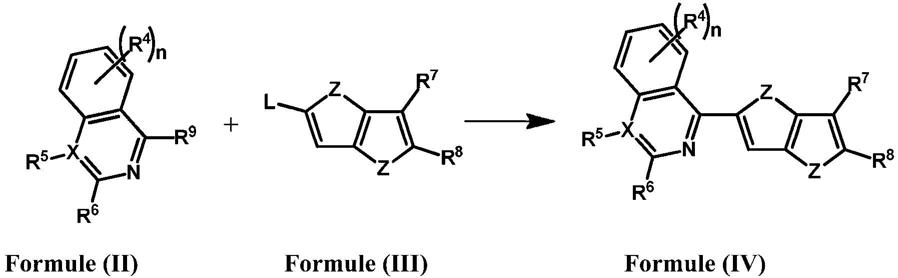
(ii) réaction du composé selon la Formule (IV) avec W₂M(R¹⁰)₄ pour obtenir un composé selon la Formule (V) 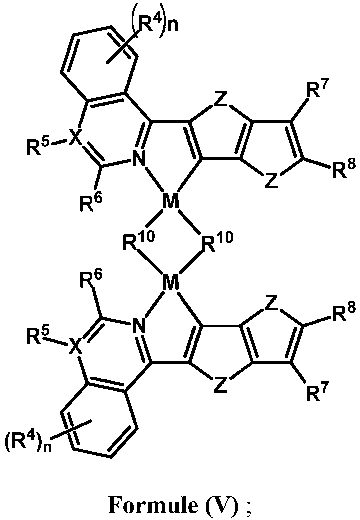 et
(iii) réaction d'un composé selon la Formule (V) avec un composé selon la Formule (VI) 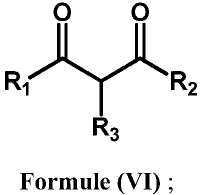
où M, R¹, R², R³, R⁴, R⁵, R⁶, R⁷, R⁸, n, X et Z sont tels que définis dans l'une quelconque des revendications 1 à 3 ;
R⁹ est un halogène ;
R¹⁰ est un halogène ; et
W est un métal alcalin.

7. Nanoparticule polymère chargée d'un composé selon la Formule (I) tel que défini dans l'une quelconque des revendications 1 à 3, ou d'un composé selon la Formule (Ia) tel que défini dans la revendication 4, ou d'un composé selon la Formule (Ib) tel que défini dans la revendication 5.

8. Nanoparticule polymère selon la revendication 7, ladite nanoparticule polymère étant un support nanolipidique, tel qu'un conjugué PEG-lipide.

9. Nanoparticule polymère selon la revendication 8, dans laquelle le poids moléculaire du PEG dans le conjugué PEG-lipide est d'environ 1000 à environ 10 000, d'environ 1000 à environ 6000, ou d'environ 2000 à environ 5000.

10. Nanoparticule polymère selon la revendication 8, dans laquelle le lipide du conjugué PEG-lipide est un phospholipide, tel que la 1,2-distéaroyl-sn-glycéro-3-phosphoéthanolamine (DSPE).

11. Nanoparticule polymère selon l'une quelconque des revendications 7 à 10, dans laquelle le composé selon la Formule (I) est compris en une quantité d'environ 0,1 % p/p à environ 10 % p/p de la teneur totale en matières solides de la nanoparticule.

12. Composition aqueuse comprenant une pluralité de nanoparticules polymères selon l'une quelconque des revendications 7 à 11.

13. Utilisation d'un composé selon la Formule (I) tel que défini dans l'une quelconque des revendications 1 à 5, ou d'une nanoparticule polymère telle que définie dans l'une quelconque des revendications 7 à 11, ou d'une composition aqueuse telle que définie dans la revendication 12, pour déterminer la concentration en oxygène dans un milieu.

14. Procédé de mesure de la concentration en oxygène dans un milieu comprenant l'utilisation d'un composé selon la Formule (I) tel que défini dans l'une quelconque des revendications 1 à 5, ou d'une nanoparticule polymère telle que définie dans l'une quelconque des revendications 7 à 11, ou d'une composition aqueuse selon la revendication 12.

15. Kit de pièces comprenant ;
a. une solution comprenant un composé selon la Formule (I) tel que défini dans l'une quelconque des revendications 1 à 5, ou une nanoparticule polymère telle que définie dans l'une quelconque des revendications 7 à 11, ou une composition aqueuse selon la revendication 12 ; et
b. des instructions d'utilisation du kit dans le procédé selon la revendication 14.
